(19) Europäisches Patentamt
European Patent Office
Office européen des brevets

(11) **EP 2 578 163 A1**

(12) **EUROPEAN PATENT APPLICATION**
published in accordance with Art. 153(4) EPC

(43) Date of publication:
**10.04.2013 Bulletin 2013/15**

(51) Int Cl.:
**A61B 8/08** (2006.01)     **A61B 8/02** (2006.01)

(21) Application number: **11792127.0**

(22) Date of filing: **03.06.2011**

(86) International application number:
**PCT/JP2011/003156**

(87) International publication number:
**WO 2011/155168 (15.12.2011 Gazette 2011/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.04.2011   JP 2011085718
07.06.2010   JP 2010130439**

(71) Applicant: **Panasonic Corporation
Kadoma-shi
Osaka 571-8501 (JP)**

(72) Inventors:
• **FAN, Shu Feng
Tai Seng Industrial Estate
Singapore 534415 (SG)**

• **TRAN, Anh Tuan
Tai Seng Industrial Estate
Singapore 534415 (SG)**
• **CHONG, Kok Seng
Tai Seng Industrial Estate
Singapore 534415 (SG)**

(74) Representative: **Kügele, Bernhard
Novagraaf International SA
Chemin de l'Echo 3
1213 Onex (CH)**

(54) **MALIGNANT TISSUE TUMOR DETECTION METHOD AND MALIGNANT TISSUE TUMOR DETECTION DEVICE**

(57)     To detect a malignant tumor more accurately by detecting a feature of a cancerous tumor pulsation more appropriately, a tissue malignant tumor detection method according to the present invention includes: segmenting, into blocks, a region scanned with ultrasound (S100); estimating, based on a scan signal, a tissue pulsation that is a temporal variation in tissue displacement derived from pulsations of a tissue (S101); extracting, for each of the blocks, pulsation-related features that are parameters related to the tissue pulsation (S103); calculating distribution characteristics of the pulsation-related features for each of the blocks (S104); and classifying, based on the distribution characteristics, whether or not each of the blocks is a malignant block that is a block including the malignant tumor (S105).

FIG. 2

```
        Start
          │
          ▼
  Segment scan region ─────── S100
          │
          ▼
  Estimate tissue pulsation ─── S101
          │
          ▼
  Extract pulsation-related features ─── S103
          │
          ▼
  Calculate distribution characteristics
  of pulsation-related features ─── S104
          │
          ▼
  Classify according to malignancy ─── S105
          │
          ▼
         End
```

EP 2 578 163 A1

## Description

[Technical Field]

The present invention relates to tissue malignant tumor detection methods and the like, and relates particularly to a tissue malignant tumor detection method and the like using ultrasound.

[Background Art]

Conventionally, many techniques have conventionally been developed to detect tissue cancerous tumors (for example, see Patent Literatures 1 to 7). Examples of contrast agents for imaging tissue perfusion include: an iodine contrast agent for computed tomography (CT), a gadolinium chelated agent for magnetic resonance imaging (MRI), and radiopharmaceuticals for positron emission tomography (PET) (see Patent Literatures 4 and 5). CT and MRI typically detect cancerous tissues by taking advantage of rapid accumulation of such contrast agent in an extra-vascular space due to increased permeability of tumoral blood vessels. PET detects cancerous tissues by increased uptake of glucose molecules due to increased metabolic demands of tumor cells. Ultrasound contract agents use larger, typically gas-filled bubbles, to allow imaging of tumoral microcirculation.

However, these methods require invasive injection of the contrast agent. Furthermore, these methods only rely on the morphological structure of microcirculation at the cancerous tumor site, and do not use the functional features of microvessels, for example, blood flow and pulsations.

Thus, use of ultrasound Doppler imaging is known as another method for detecting cancerous tumors from the blood perfusion of the tumors themselves without use of the invasive injection of the contrast agent (although the use of the contrast agent in ultrasound Doppler imaging is also very common).

Patent Literature 1 discloses a system that detects an early prostate cancer by combining the tissue density and comparison of blood flow.

Patent Literature 2 discloses a method of using Power Doppler imaging to calculate the degree of neo-microvasculation in a tumor so as to detect the degree of malignancy and metastatic ability of the cancerous tumor whose location is fully known.

Patent Literature 3 discloses a method for detecting tissue abnormality by comparing the blood perfusion in the tissue before and after applying a heat source, based on an assumption that tissue blood perfusion increases with heat.

[Citation List]

[Patent Literature]

[PTL 1] US Patent Application Publication No. 2003/0149358, Specification
[PTL 2] US Patent Application Publication No. 2006/0241463, Specification
[PTL 3] US Patent No. 5,233,994, Specification
[PTL 4] US Patent No. 4,276,885, International Publication No. 80/02365
[PTL 5] International Publication No. 01/28594
[PTL 6] US Patent Application Publication No. 2004/0199077, Specification; US Patent No. 6984,211, Specification
[PTL 7] US Patent Application Publication No. 2005/0027188, Specification; US Patent No. 7,466,848, Specification

[Summary of Invention]

[Technical Problem]

However, all of these methods only use Doppler imaging to measure the blood velocity, and do not use, for example, characteristics of a tissue pulsation waveform (hereinafter, also referred to as a pulsation curve) in time domain. In other words, these methods use only part of angiogenesis-related information obtained from the tissues.

Tumor angiogenesis creates new blood vessels having different structures from those of normal tissues. These new vessels associated with cancerous tumors have characteristics such as perivascular detachment, vessel dilation, and irregular shape. Tumoral blood vessels do not have smooth muscle cells which exist in the blood vessels in normal tissues and are not formed sufficiently to provide oxygen to all the tissues. Furthermore, tumoral blood vessels are more porous and leaky than blood vessels in normal tissues.

However, the conventional techniques attempt to detect the pulsations of cancerous tumors only by measurement using Doppler imaging, and do not disclose any detection method that makes use of a feature quantity having the characteristics of cancerous tumor pulsations as described above.

Thus, an object of the present invention is to provide a tissue malignant tumor detection method for detecting a malignant tumor more accurately by detecting the characteristics of cancerous tumor pulsations more appropriately.

[Solution to Problem]

A tissue malignant tumor detection method according to an aspect of the present invention is a tissue malignant tumor detection method for detecting a malignant tumor included in a tissue, using a scan signal obtained by scanning the tissue with ultrasound, and the tissue malignant tumor detection method includes: segmenting a scanned region of the tissue into a plurality of blocks; estimating a tissue pulsation for each of the blocks, based on the scan signal, the tissue pulsation being a temporal variation in displacement of the tissue caused by pulsation of the tissue; extracting a plurality of pulsation-related features for each of the blocks, the pulsation-related features being parameters related to the tissue pulsation; calculating distribution characteristics of the pulsation-related features for each of the blocks; and classifying, based on the distribution characteristics, whether or not each of the blocks is a malignant block that is a block including a malignant tumor.

With this, it is possible to calculate, for each of the blocks included in the scanned region, a plurality of feature quantities each of which corresponds to a plurality of influences of malignant tumor angiogenesis on tissue pulsations, from the information indicating temporal variation in tissue displacement. From the feature quantities thus calculated, the blocks included in the scanned region can be classified into a malignant block having a high probability of including a malignant tumor and the other blocks. As a result, it is possible to detect the malignant tumor more accurately by detecting the characteristics of cancerous tumor pulsations more appropriately.

Note that the present invention can be realized not only as such a tissue malignant tumor detection method but also as a tissue malignant tumor detection apparatus, and also as a program for causing a computer to execute characteristic steps included in the tissue malignant tumor detection method. In addition, it goes without saying that such a program can be distributed through a recording medium such as a compact disc read only memory (CD-ROM) and a transmission medium such as the Internet.

Furthermore, the present invention can be realized as a semiconductor integrated circuit (LSI) which realizes part or all of functions of such a tissue malignant tumor detection apparatus, and can also be realized as a tissue malignant tumor detection system including such a tissue malignant tumor detection apparatus.

[Advantageous Effects of Invention]

It is possible to provide a tissue malignant tumor detection method which detects a malignant tumor more accurately by detecting characteristics of cancerous tumor pulsations more appropriately.

[Brief Description of Drawings]

[Fig. 1] Fig. 1 is a diagram showing a functional block of a tissue malignant tumor detection apparatus according to an embodiment of the present invention.
[Fig. 2] Fig. 2 is a flowchart showing a processing flow of the tissue malignant tumor detection apparatus according to the embodiment of the present invention.
[Fig. 3] Fig. 3 is a diagram showing an example of a configuration of a tissue pulsation estimation unit according to the embodiment of the present invention.
[Fig. 4] Fig. 4 is a diagram showing an example of specifications of ultrasonic source data according to the embodiment of the present invention.
[Fig. 5] Fig. 5 is a diagram showing an example of a configuration of a pre-processing unit according to the embodiment of the present invention.
[Fig. 6] Fig. 6 is a diagram showing an example of a configuration of a major component extraction unit according to the embodiment of the present invention.
[Fig. 7] Fig. 7 is a diagram showing an example of a result of processing performed by an extrema identification unit and a pulsation adjustment unit according to the embodiment of the present invention.
[Fig. 8] Fig. 8 is a diagram showing an example of a configuration of an amplitude feature calculation unit according to the embodiment of the present invention.
[Fig. 9] Fig. 9 is a diagram showing an example of a configuration of a shape feature calculation unit according to the embodiment of the present invention.

[Fig. 10] Fig. 10 is a diagram showing an example of a configuration of a cardiac cycle identification unit according to the embodiment of the present invention.

[Fig. 11] Fig. 11 is a diagram showing an example of processing performed by a critical point identification unit according to the embodiment of the present invention.

[Fig. 12] Fig. 12 is a diagram showing an example of a pulsation shape feature of a cardiac pulsation according to the embodiment of the present invention.

[Fig. 13] Fig. 13 is a diagram showing a comparison in pulsation shape feature between a tumoral tissue pulsation and a normal tissue pulsation.

[Fig. 14] Fig. 14 is a diagram showing an example of the configuration of a major comportment extraction unit according to the embodiment of the present invention.

[Fig. 15] Fig. 15 is a diagram showing an example of a result of each process of segmentation, tapering, and representative extraction that is performed on a signal in the major comportment extraction unit according to the present invention.

[Fig. 16] Fig. 16 is a diagram showing an example of a configuration of a spatial distribution calculation unit according to the embodiment of the present invention.

[Fig. 17] Fig. 17 is a diagram showing an example of processing of gray-level co-occurrence matrix calculation according to the embodiment of the present invention.

[Fig. 18] Fig. 18 is a diagram showing an example of a configuration of a tumor localization unit according to the embodiment of the present invention.

[Fig. 19] Fig. 19 is an example of processing performed by the tumor localization unit according to the embodiment of the present invention.

[Fig. 20] Fig. 20 is a diagram showing an example of a configuration of a cardiac cycle delay calculation unit according to the embodiment of the present invention.

[Fig. 21] Fig. 21 is a diagram showing an example of a configuration of a delay calculation unit according to the embodiment of the present invention.

[Fig. 22] Fig. 22 is a diagram showing an example of a configuration of an autoregressive coefficient calculation unit according to the embodiment of the present invention.

[Fig. 23] Fig. 23 is a diagram showing an example of a result in the case where no tumor is included in a tissue, which is output from the tissue malignant tumor detection apparatus according to the embodiment of the present invention.

[Fig. 24] Fig. 24 is a diagram showing an example of a result in the case where a tumor is included in a tissue, which is output from the tissue malignant tumor detection apparatus according to the embodiment of the present invention.

[Fig. 25] Fig. 25 is a block diagram showing a hardware configuration of a computer system which realizes the tissue malignant tumor detection apparatus according to the embodiment of the present invention.

[Description of Embodiment]

[Embodiment]

A tissue malignant tumor detection method according to an aspect of the present invention is a tissue malignant tumor detection method for detecting a malignant tumor included in a tissue, using a scan signal obtained by scanning the tissue with ultrasound, and the tissue malignant tumor detection method includes: segmenting a scanned region of the tissue into a plurality of blocks; estimating a tissue pulsation for each of the blocks, based on the scan signal, the tissue pulsation being a temporal variation in displacement of the tissue caused by pulsation of the tissue; extracting a plurality of pulsation-related features for each of the blocks, the pulsation-related features being parameters related to the tissue pulsation; calculating distribution characteristics of the pulsation-related features for each of the blocks; and classifying, based on the distribution characteristics, whether or not each of the blocks is a malignant block that is a block including a malignant tumor.

With this, it is possible to calculate, for each of the blocks included in the scanned region, a plurality of feature quantities each of which corresponds to a plurality of influences of malignant tumor angiogenesis on tissue pulsations, from the information indicating variation in tissue displacement with time. From the feature quantities thus calculated, the blocks included in the scanned region can be classified into a malignant block having high probability of including a malignant tumor and the others. As a result, it is possible to detect a malignant tumor more accurately by detecting the characteristics of cancerous tumor pulsations more appropriately.

In addition, the tissue malignant tumor detection method may further include locating a position of the malignant tumor, based on a block classified as the malignant block in the classifying.

With this, it is possible to locate the malignant tumor accurately by associating the malignant block with the tissue location.

Specifically, the estimating may include: calculating a tissue displacement using the scan signal, the tissue displacement being a displacement of the tissue in spatial position; calculating tissue skewness from the calculated tissue displacement, the tissue skewness being a spatial gradient of the tissue displacement; and generating a pulsation waveform that is a waveform of the tissue pulsation and indicates the tissue displacement versus time or the tissue skewness versus time.

In addition, the tissue malignant tumor detection method may further include extracting, from the estimated tissue pulsation, a component due to a cardiac pulsation derived from pulsations of a heart.

With this, it is possible to eliminate an interference component that is noise at the time of sensing with ultrasound, to detect the malignant tumor more accurately.

Specifically, the extracting of a component may further include: calculating, of the estimated tissue pulsation, cardiac power that is electric power related to the cardiac pulsation; clustering the scanned region of the tissue, based on a magnitude of the cardiac power; identifying an extremum of an amplitude of the cardiac pulsation in the scanned region of the tissue that is clustered; and adjusting an amplitude of a pulsation waveform that is a waveform of the tissue pulsation, based on the extremum.

In addition, the identifying of an extremum may further include: identifying peaks and troughs in the pulsation waveform; and rejecting an outlier in the amplitude of the pulsation waveform, so as to eliminate an interference peak and an interference trough, the interference peak being a peak with which a gap from another peak is larger than a predetermined threshold, and the interference trough being a trough with which a gap from another trough is larger than a predetermined threshold.

With this, it is possible to detect the malignant tumor more accurately, from the data that does not include an outlier.

In addition, the extracting of a component may further include: eliminating portions related to an interference peak and an interference trough, the interference peak being a peak with which a gap from another peak is larger than a predetermined threshold, and an interference trough being a trough with which a gap from another trough is larger than a predetermined threshold; and adjusting the amplitude of the pulsation waveform so that the peaks in the pulsation waveform are aligned in a temporal direction and the troughs in the pulsation waveform are aligned in the temporal direction.

With this, it is possible to calculate the feature quantities more accurately by aligning amplitudes of the pulsation waveform.

In addition, in the estimating, the tissue pulsation may be estimated for all scan points in each of the blocks.

In addition, in the estimating, the tissue pulsation may be estimated as at least one representative pulsation for each of the blocks or a combination of the at least one representative pulsation.

With this, it is possible to determine whether or not the malignant tumor is present, by comprehensive determination using the data obtained from the scan points.

In addition, the pulsation-related features may include at least one of: a pulsation amplitude feature that is a feature quantity related to an amplitude of the tissue pulsation; a pulsation shape feature that is a feature quantity related to a shape of a waveform of the tissue pulsation; and a pulsation temporal feature that is a feature quantity related to a temporal variation of the waveform of the tissue pulsation.

With this, it is possible to perform more accurate determination, based on the feature quantities, such as the amplitude, shape, and temporal change of the pulsation waveform.

More specifically, the pulsation shape feature may be at least one of: L1/L2 which is a ratio between L1 that is a systolic duration and L2 that is a diastolic duration, the systolic duration being a duration of a systolic part of a cardiac cycle, and the diastolic duration being a duration of a diastolic part of the cardiac cycle; L3/L4 which is a ratio between L3 that is a period from a systolic mid-point to a diastolic mid-point and L4 that is a duration of a cardiac pulsation, the systolic mid-point being a point at which, on a systolic curve, a ratio of the amplitude with respect to a maximum amplitude equals a predetermined ratio in the systolic duration, and the diastolic mid-point being a point at which, on a diastolic curve, a ratio of the amplitude with respect to the maximum amplitude equals a predetermined ratio in the diastolic duration; a deviation of the diastolic curve from a predetermined curve connecting a systolic peak that is a peak in the amplitude in the systolic duration and a diastolic ending point that is an ending point of the diastolic duration; skewness which represents asymmetry of the cardiac pulsation; kurtosis which represents peakedness of the systolic peak; and a deviation of an extremum included in the diastolic curve.

In addition, the pulsation shape feature may be calculated in: calculating a cardiac cycle from the tissue pulsation; identifying a critical point using the cardiac cycle; and extracting the pulsation shape feature based on the cardiac cycle and the critical point.

In addition, the critical point of the cardiac cycle may include: a systolic starting point which is a starting point of a systolic part of the cardiac cycle; a diastolic ending point which is an ending point of a diastolic part of the cardiac cycle; a systolic peak which is a peak in the amplitude during the systolic part; a systolic mid-point which is a point at which, on a systolic curve, a ratio of the amplitude with respect to a maximum amplitude equals a predetermined ratio during the systolic part of the cardiac cycle; and a diastolic mid-point which is a point at which, on a diastolic curve, a ratio of the amplitude with respect to a maximum amplitude equals a predetermined ratio during the diastolic part of the cardiac cycle.

In addition, the identifying of a critical point may include: searching a minimum point and a maximum point in the tissue pulsation; identifying a pulsation direction which indicates whether the tissue pulsation has an upward systolic curve or a downward systolic curve, based on the minimum point and the maximum point; and determining the critical point in the pulsation waveform, using the maximum point, the minimum point, and the pulsation direction.

In addition, the predetermined curve may be linear.

In addition, in the extracting of pulsation-related features, the deviation may be calculated as a sum of positive differences between the diastolic curve and the predetermined curve when the pulsation has an upward systolic curve, and the deviation may be calculated as a sum of negative differences between the diastolic curve and the predetermined curve when the pulsation has a downward systolic curve.

With this, it is possible to perform more accurate determination by calculating the feature quantity that differs depending on whether the pulsation is in an expansion period or in a contraction period.

In addition, the pulsation temporal feature may be at least one of: a delay of a cardiac cycle; a difference in a cardiac waveform that is a waveform of a cardiac pulsation; and an autoregressive coefficient of a waveform of the tissue pulsation.

Specifically, the delay of the cardiac cycle may be calculated in calculating of the delay of the cardiac cycle, which includes: determining a reference cardiac cycle that is a cardiac cycle to be referenced; and calculating a delay of a target cardiac cycle with respect to the reference cardiac cycle.

More specifically, in the determining of a reference cardiac cycle, a cardiac cycle having a largest amplitude among scan data, may be selected as the reference cardiac cycle.

In addition, the reference cardiac cycle may be determined from an electrocardiography (ECG) signal.

In addition, the difference in the cardiac waveform may be calculated in the calculating of a delay of a target cardiac cycle, which includes: calculating a reference cardiac waveform that is a cardiac waveform to be referenced; calculating a difference between each of a plurality of cardiac waveforms derived from the pulsation and the reference cardiac waveform; and calculating a total cardiac waveform difference value from a plurality of differences each of which is the calculated difference, the total cardiac waveform difference value being a value representing the differences between the cardiac waveforms and the reference cardiac waveform.

In addition, the total cardiac waveform difference value may be a standard deviation of the calculated differences.

In addition, the autoregressive coefficient may be calculated in calculating the autoregressive coefficient, which includes: tapering a plurality of pulsation waveforms to have the same cardiac cycle; and calculating the autoregressive coefficient which is a coefficient of a predetermined autoregressive model, based on the predetermined autoregressive model and the tapered pulsation waveforms.

In addition, the distribution characteristics may be at least one of a spatial distribution parameter and a statistical distribution parameter.

With this, it is possible to perform more accurate judgment using a distribution which considers a spatial locational relationship and a distribution which considers statistical characteristics.

Specifically, the spatial distribution parameter may be at least one of energy, entropy, contrast, homogeneity, and correlation of the pulsation-related features.

In addition, the statistical distribution parameter may be at least one of a mean, a median, a largest value, a smallest value, a standard deviation, kurtosis, and skewness of the pulsation-related features.

In addition, the pulsation-related features and the distribution characteristics of the pulsation-related features may include at least one of: a median, entropy, a standard deviation, a mean, and a largest value of the pulsation amplitude at a plurality of scan points in each of the blocks; a median, entropy, a standard deviation, a mean, and a largest value of a cardiac waveform difference at the scan points in each of the blocks; a median of a ratio between a systolic duration and a diastolic duration at the scan points in each of the blocks, the systolic duration being a duration of a systolic part of a cardiac cycle, and the diastolic duration is being a duration of a diastolic part of the cardiac cycle; a ratio between the systolic duration and the diastolic duration of a representative pulsation in each of the blocks; and a largest deviation value and a standard deviation of a diastolic curve at the scan points in each of the blocks.

In addition, the locating may further include: setting a target region for each of scan points in the scanned tissue; identifying a block belonging to the target region, from among the blocks; and calculating a probability of the tissue being a cancer, based on a result of the classifying performed on the block belonging to the target region.

In addition, the locating may further include: displaying, in two or three dimensional image, the probability of the tissue being a cancer at the scan points in the scanned tissue.

A tissue malignant tumor detection apparatus according to an another aspect of the present invention is a tissue malignant tumor detection apparatus which detects a malignant tumor included in a tissue, using a scan signal obtained by scanning the tissue with ultrasound, and the tissue malignant tumor detection apparatus includes: a block segmentation unit which segments a scanned region of the tissue into a plurality of blocks; a tissue pulsation estimation unit which estimates a tissue pulsation for each of the blocks based on the scan signal, the tissue pulsation being a temporal variation in displacement of the tissue caused by pulsation of the tissue; a pulsation-related feature extraction unit which extracts pulsation-related features for each of the blocks, the pulsation-related features being parameters related to the tissue pulsation; a distribution characteristics calculation unit which calculates distribution characteristics of the pulsation-related features for each of the blocks; and a malignancy classification unit which classifies, based on the distribution characteristics, whether or not each of the blocks is a malignant block that is a block including a malignant tumor.

With this configuration, it is possible to detect the features of the cancerous tumor pulsation more appropriately, from the feature quantities. As a result, it is possible to provide a tissue malignant tumor detection apparatus which can determine the malignant tumor more accurately.

In addition, the tissue malignant tumor detection apparatus may further include a tumor localization unit which locates a position of the malignant tumor, based on a block classified as the malignant block by the malignancy classification unit.

With this, it is possible to locate the malignant tumor with ease and accuracy.

As a premise of the present invention described above, cancerous tumor angiogenesis and cancerous tumor pulsation generated thereby will be described in detail.

Cancerous tumors require creation of new blood vessels to supply nutrients and oxygen to themselves and remove waste products generated as a result of metabolism. Such new blood vessel creation process is called angiogenesis. It is well known that an early stage cancerous tumor requires creation of blood vessels to grow beyond approximately 2 to 3 millimeters in diameter. These new blood vessels grow surrounding and inside the tumor, and with the increase of blood vessels, cancer cells have access to main vessels in the body, which accordingly increases a change of metastasis through the newly created blood vessels. Detection of angiogenesis may lead to the detection of the early stage cancerous tumor, and even to a prediction of metastatic probability after treatment.

Here, cancerous tumors (also referred to as malignant tumors) are different from normal tissues or benign tumors in many aspects.

First, angiogenesis occurs at the level of capillary bed. Pulsations in tissues are created by the heart pumping the blood to various parts of the body, which causes the tissues to pulsate through the blood perfusion. With the increase in capillary blood supply, it is assumed that the pulsation amplitude of the tumor increases compared to the surrounding normal tissues. At the same time, it is also assumed that the blood flowing in and out of the cancerous tumor also increases.

The new vessels associated with cancerous tumors have characteristics such as perivascular detachment, vessel dilation, and irregular shape. Tumoral blood vessels do not have smooth muscle cells which exist in the blood vessels in normal tissues and are not formed sufficiently to provide oxygen to all the tissues. Furthermore, tumoral blood vessels are more porous and leaky than blood vessels in normal tissues.

All these factors may cause irregularity, in tumors, in pulse arrival time, pulse waveform shape, pulse amplitude variation with respiration, and baseline variations with respiration, which are different from regular variations of normal tissues. Because difference in spatial location causes difference in pulsation pattern, it is assumed that the cancerous tumor pulsation waveform has a dilated shape than that of normal tissues.

This difference in microvascular system also causes difference in appearance timing of the pulsation waveform, and represents temporal variation characteristics. In addition, the difference is also difference in distribution characteristics of the pulsation statistically and spatially represented.

Accordingly, by discriminating the difference in pulsation detected from the tissue as a result of blood perfusion, it is possible to classify between cancerous and normal tissues.

Specifically, in the embodiment of the present invention, to allow determining the malignancy of a scanned tissue, main parameters from the pulsation, that is, (1) pulsation amplitude, (2) pulsation shape, (3) pulsation temporal feature, and (4) distribution characteristics thereof are used as feature quantities.

When angiogenesis causes increase in capillary blood supply and microvascular system, there is a possibility that the pulsation amplitude differs between cancerous tissues and normal tissues.

The tumoral blood vessels are more porous and leaky than those in normal tissue, and such cancerous microvessels have no smooth muscles, thus causing such vessels to be in a vasodilation state. This is likely to cause a difference in pulsation shape, which is measured by the features extracted from the pulsation waveform, that is, L1/L2, L3/L4, skewness, kurtosis, the deviation of a diastolic curve, and a deviation of extrema in diastolic curve. These will be further described in the embodiment.

Note that L1/L2 is a ratio between a systolic duration (L1) and a diastolic duration (L2), and L1/L2 and skewness represent how delayed the systolic duration is. The more dilated the systolic duration, the higher the probability of the pulsation being derived from the cancerous tumor.

L3/L4 is, for example, a ratio between a pulsation width at half height (L3) and an overall pulsation period (L4), and L3/L4 represents how wide and dilated the pulsation waveform is. The more dilated the pulsation waveform, the higher the probability of being cancerous pulsation.

Then, the deviation of the diastolic curve and the deviation of the extrema in the diastolic curve indicate whether or not a dicrotic notch is included in the pulsation waveform and curvature of the diastolic curve. Lack of the dicrotic notch indicates the possibility of being cancerous pulsation.

The pulsation temporal feature is a feature quantity related to temporal change in pulsation waveform, which describes how the pulsation changes with time. Specifically, the pulsation temporal feature indicates: a delay of a cardiac cycle (also referred to as a cardiac period); a cardiac waveform difference; and an autoregressive coefficient.

Furthermore, the spatial and statistical characteristics of the above features in a specific tissue region are also effective in representing an underlying pulsation distribution resulting from the difference in microvascular structures, and represents how the pulsations are distributed in the region.

These features can be selected and used to classify between malignant and benign tissues.

Then, results of the classification are combined to produce malignant scores of a region inside the scanned tissue, and these scores indicate to a doctor or a patent, the probability of a cancerous tumor existing in the scanned tissue.

Thus far, the premise of the present invention and an outline of an example of the feature quantities according to the present invention have been described. Next, an embodiment of the tissue malignant tumor detection apparatus and the tissue malignant tumor detection method according to the present invention will be described with reference to the drawings. Note that the following embodiment merely describes a principle of various inventive steps. It is understood that many variations of specific examples described herein are obvious to those skilled in the art.

The method described in the present invention is represented as steps and advantageous effects, occasionally using two-dimensional data. However, these data are merely intended for illustration, and any implementation of this kind using three-dimensional data would be obvious to those skilled in the art. Such implementations using the three-dimensional data are also within the scope of the present invention.

A medical imaging device may include some or all of the following components: a signal transmission and receiving device, a data acquisition component, a data processing component, and a display component. The present invention is to teach a method performed in the data processing component of the medical imaging device. An input to such a data processing component is a scan signal obtained from the data acquisition component, and an output is shown to a user through the display component. It is understood that another component of such a medical imaging device is applicable to an application example of the present invention.

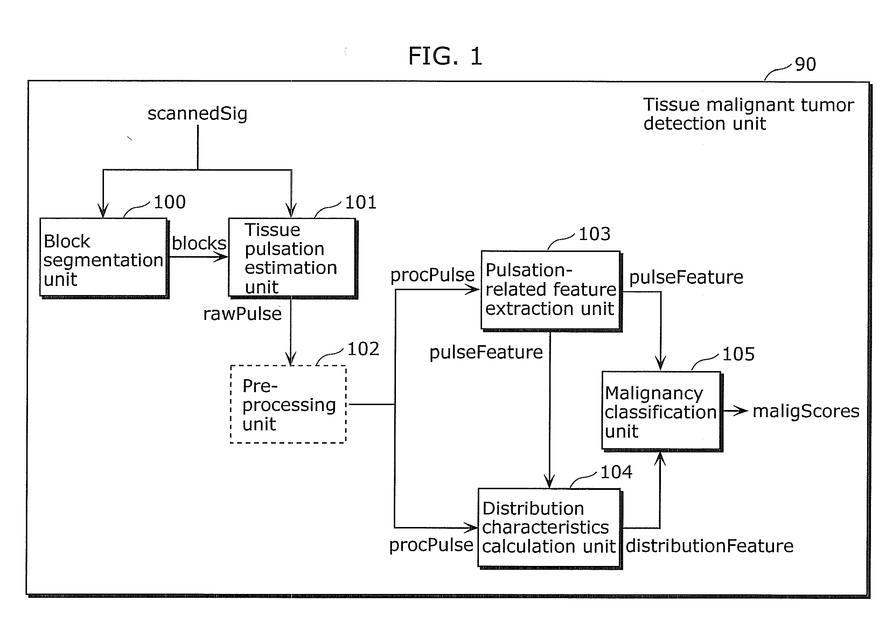

Fig. 1 shows a main embodiment of the present invention. Fig. 1 is a diagram showing a functional block of a tissue malignant tumor detection apparatus 90 according to the embodiment of the present invention.

Here, scannedSig represents scan data received from a diagnostic imaging device used to detect the pulsations in the scanned tissue. An example of such a device is a medical ultrasound device, in which scannedSig is either radio-frequency (RF) data or IQ data which represents a sinusoidal amplitude and a phase variation that are obtained by demodulating the RF data. The basic operation of the ultrasound device is to transmit a radio frequency ultrasound pulse toward a medium to be scanned, and, as a result of the pulse subsequently interacting with the underlying structure along its path while being reflected and scattered, receive a reflected and scattered signal. In other words, the scannedSig is a signal representing a reflected wave of the ultrasound pulse transmitted toward the tissue to be scanned. Using such signals, it is possible to estimate the morphological and functional characteristics of the underlying structure. One of the advantages of using ultrasound as a medical imaging technique is ability to generate a high-frame rate image and detect small movements in the tissue.

According to the main embodiment as shown in Fig. 1, the tissue malignant tumor detection apparatus 90 is a tissue malignant tumor detection apparatus which detects a malignant tumor included in a tissue, using a scan signal obtained by scanning the tissue with ultrasound, and more specifically, the tissue malignant tumor detection apparatus 90 includes: a block segmentation unit 100, a tissue pulsation estimation unit 101, a pre-processing unit 102, a pulsation-related feature extraction unit 103, a distribution characteristics calculation unit 104, and a malignancy classification unit 105. Note that the pre-processing unit 102 is optional and is included for the illustrative purpose. Implementations without the pre-processing unit 102 are still within the scope of the present invention.

The block segmentation unit 100 represents a process of segmenting a scanned region that is a region where tissues are scanned with ultrasound, into tissue blocks having a specific shape and size (hereinafter referred to as blocks). The shape of the blocks can be selected to be square, rectangular, polygonal, or circular but is not only limited to these shapes. The size can be, but not limited to, 1 millimeter.

The tissue pulsation estimation unit 101 estimates a fundamental pulsation of the scanned tissue (hereinafter referred to as tissue pulsation) from the received scannedSig. Specifically, the tissue pulsation estimation unit 101 estimates, for each of the blocks, the tissue pulsation that represents temporal variation in tissue displacement caused by the tissue pulsation, based on the scan signal. Here, it is assumed that each block includes at least one scan signal.

An output of the tissue pulsation estimation unit 101 is rawPulse that is a signal representing an unprocessed tissue pulsation. Possible parameters to represent the pulsations in the tissue include, but not limited to, tissue displacement and tissue skewness. Tissue displacement is estimated as an absolute movement of the tissue according to an original location of the tissue at time zero. Tissue skewness is estimated as a relative displacement between parts within the tissue. In other words, tissue skewness is a spatial gradient of tissue displacement. It is possible to select either tissue displacement versus time or tissue skewness versus time at a specific tissue point (that is, the scan signal obtained from a certain point in the tissue to be scanned), to represent the tissue pulsation at the point. In other words, tissue pulsation is a temporal variation of displacement at a spatial position in the tissue. Note that the tissue pulsation may include both tissue skewness and tissue displacement.

The pre-processing unit 102 eliminates interference (what is called noise) in the estimated raw pulsation. The unprocessed tissue pulsation, in some cases, includes various types of tissue movements. One of such movements is cardiac pulsation as a result of blood flowing through blood vessels according to a cardiac cycle of the heart. Another movement is a respiration cycle of a patient, which is several times slower than the cardiac cycle and usually has amplitude significantly larger than the cardiac pulsation. This movement is not important in the present invention. Other environmental and electronic interferences also occur in some cases. The pre-processing unit 102 outputs clean pulsation with suppressed interference, that is, procPulse. Note that in the case where the rawPulse output from tissue pulsation estimation unit 101 includes small interferences, the tissue malignant tumor detection apparatus 90 produces the same advantageous effect even if the pre-processing unit 102 is not included. For example, when a subject to be scanned is at rest, it is possible to extract the features using the pulsation-related feature extraction unit 103 even if the pre-processing unit 102 is not included. However, it is possible to detect the malignant tumor more accurately when the pre-processing unit 102 is included.

The pulsation-related feature extraction unit 103 performs calculation processing, on each of the blocks, to extract different types of pulsation-related features which are parameters related to the pulsation waveform in time domain. An output of this block is pulsefeature, and is an input signal to the distribution characteristics calculation unit 104 and the malignancy classification unit 105.

The distribution characteristics calculation unit 104 calculates a parameter related to representative distribution characteristics, among the extracted characteristics, of each block. An output of this distribution characteristics calculation unit 104 is distributionFeature, and is also an input to the malignancy classification unit 105.

The malignancy classification unit 105 calculates information necessary for classifying whether the regions in the tissue are benign or malignant, and classifies each of the blocks whether or not the block is a malignant block including a malignant tumor. An output of this malignancy classification unit 105 is maligScores.

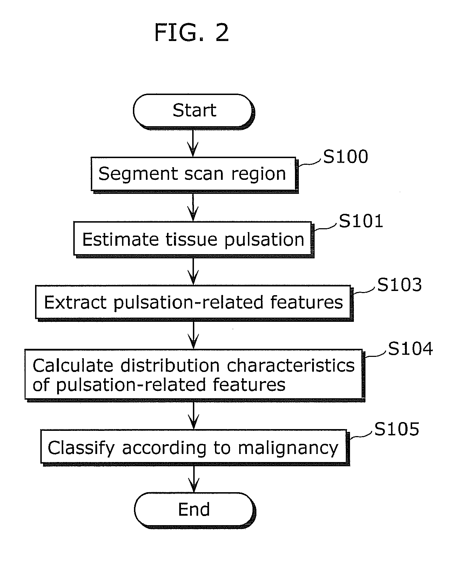

The processing flow of the tissue malignant tumor detection apparatus 90 as described above will be described with reference to Fig. 2.

Fig. 2 is a flowchart showing a processing flow of the tissue malignant tumor detection apparatus 90.

First, upon receiving a tissue scan signal from an external device, the block segmentation unit 100 segments, into blocks, the scan region indicated by the scan signal (S100).

Next, the tissue pulsation estimation unit 101 estimates tissue pulsation for each of the blocks (S101).

Next, the pulsation-related feature extraction unit 103 extracts a pulsation-related feature that represents a feature of the tissue pulsation in time domain (S103).

Next, the distribution characteristics calculation unit 104 calculates distribution characteristics that represent how values of the pulsation-related feature are distributed for each block (S104).

Lastly, the malignancy classification unit 105 determines malignancy of a tumor located in a domain corresponding to each block in the tissue and classifies the tumor as either benign or malignant (S105).

Next, with reference to Fig .1 again, some specific examples are shown based on the main example as shown in Fig. 1.

The block segmentation unit 100 in Fig. 1 segments the region where tissues are scanned with ultrasound, into tissue blocks which are smaller regions. Further processing is all performed on these tissue blocks. The shape and size of the tissue blocks are determined by an actual application example. The shape of the blocks can be selected to be square, rectangular, polygonal, or circular but is not only limited to these shapes. The size can be selected to be 1 millimeter. The inventors have experimented to prove that a rectangular tissue block of approximately 1 to 2 millimeters is effective. An output of the block segmentation is blocks which is information representing a structure of the tissue block.

The tissue pulsation estimation unit 101 in Fig. 1 estimates the unprocessed pulsation (that is a pulsation before interference elimination processing is performed) from the tissue, using the ultrasound raw RF data that is a scan signal output as a result of scanning the tissue with ultrasound by an imaging device including an ultrasound transducer.

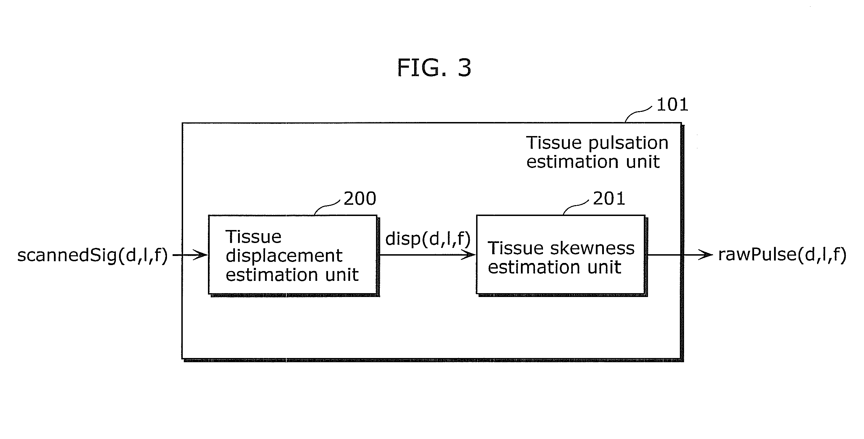

An example of the tissue pulsation estimation unit 101 is, but not limited to, a configuration as shown in Fig. 3. Fig. 3 illustrates a configuration that estimates, using ultrasound, the tissue pulsation represented by tissue skewness.

As shown in Fig. 3, the tissue pulsation estimation unit 101 according to the embodiment includes a tissue displacement estimation unit 200 and a tissue skewness estimation unit 201.

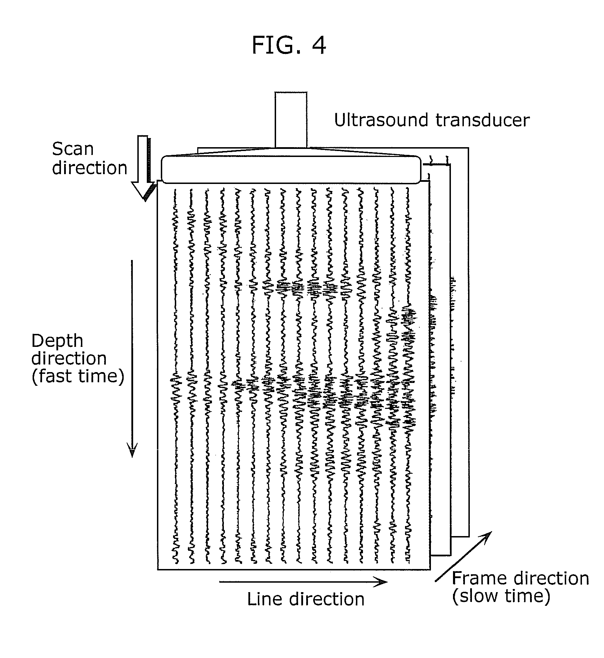

Here, with reference to Fig. 4, assuming that the scan direction by the ultrasound transducer is toward the tissue (a direction perpendicular to a body surface), the ultrasound raw RF data that is the output of the ultrasound transducer is represented by scannedSig(d, I, f). Here, d represents a depth direction parallel to the scan direction and is also referred to as fast time, I represents a line direction orthogonal to the scan direction, and f represents time or frame and is also referred to as slow time.

The tissue displacement estimation unit 200 obtains scannedSig(d, I, f) as input, and calculates disp(d, I, f) which represents tissue displacement. Various methods for calculating the displacement from the unprocessed ultrasound raw RF include, but not limited to, auto-correlation and cross-correlation.

The tissue displacement estimation unit 201 obtains, as input, disp(d, I, f) which represents tissue displacement and calculates tissue skewness. An example of methods for calculating the tissue skewness is to calculate a spatial gradient of the displacement along the depth direction, that is, a derivative of disp(d, I, f) with respect to d.

The tissue skewness calculated at a specific depth and a specific line plotted against time indicates rawPulse(d, I, f) that is a tissue pulsation at a certain tissue point. Each tissue point is described by the corresponding depth and line. Accordingly, the tissue point is described by a specific pulsation waveform at rawPulse(d, I, f). This pulsation waveform incorporates any type of movements and interferences which can occur in tissues.

The pre-processing unit 102 in Fig. 1 eliminates interference by performing further processing on the unprocessed pulsation rawPulse, thus facilitating extraction of important pulsations. The pre-processing unit 102 is optional, and implementations that do not include this block are still within the scope of the present invention. An output of the pre-processing is procPulse.

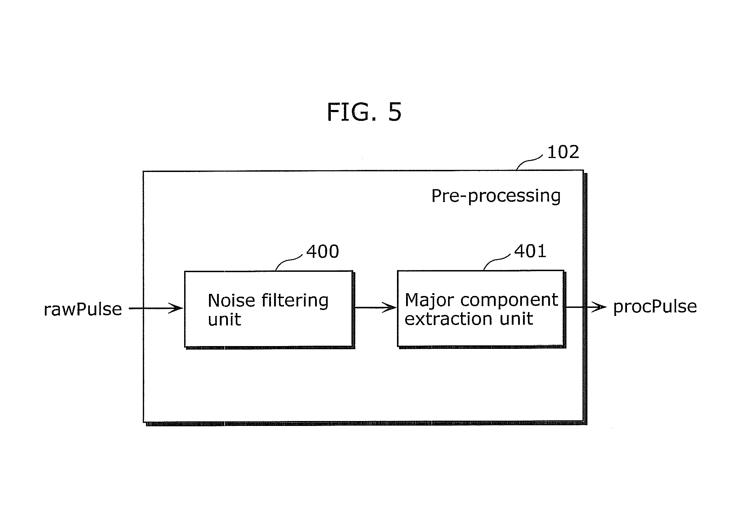

Fig. 5 shows a configuration of such a pre-processing unit 102.

As shown in Fig. 5, the pre-processing unit 102 includes a noise filtering unit 400 and a major comportment extraction unit 401.

Note that the configuration of the pre-processing unit 102 is not limited to these. This is only intended for illustration, and steps and substeps to be performed by these constituent units can be performed in any sequence and combination, any of these steps can be skipped without affecting the other steps, and any of these steps may be included in another block in Fig. 1, and such implementations are still within the scope of the present invention.

The noise filtering unit 400 is a possible constituent element included in the pre-processing unit 102. A cardiac pulsation has a frequency of 1 to 2 Hz, and a respiratory cycle has a frequency of less than 0.5 Hz. Thus, by configuring the noise filtering unit 400 as a bandpass filter which passes only cardiac pulsations having a frequency of 1 to 2 Hz, it is possible to effectively eliminate both environmental vibration noise of high frequency and respiratory movement of low frequency without affecting the characteristics of the cardiac pulsation.

The major comportment extraction unit 401, which is an example of another possible constituent element included in the pre-processing unit 102, extracts a major pulsation component in a selected region of interest (that is, the major pulsation component among pulsation components included in the tissue pulsation). This is effective when the noise filtering unit 400 cannot fully suppress interferences, particularly, irregular interferences such as body movements. The input of this block may be an unprocessed pulsation or a pulsation already processed in another pre-processing step. Likewise, the output of this block may be an input to another pre-processing step, or may be directly used as an input to the pulsation-related feature extraction unit 103 and the distribution characteristics calculation unit 104.

In one embodiment, the major comportment extraction unit 401 can extract major components using, but not limited to, Principal Component Analysis (PCA) or Independent Component Analysis (ICA). Here, the major components are cardiac pulsation and respiratory movement, and all the other components are assumed to be interferences. PCA or ICA obtains a pulsation in a predetermined region, and extracts the most dominant component in the pulsation, which is uncorrelated (in the case of PCA) or independent (in the case of ICA) with respect to the other components (that is, interferences). This is executed in exchange for reduction in spatial resolution. Thus, the major component of interest can be selected.

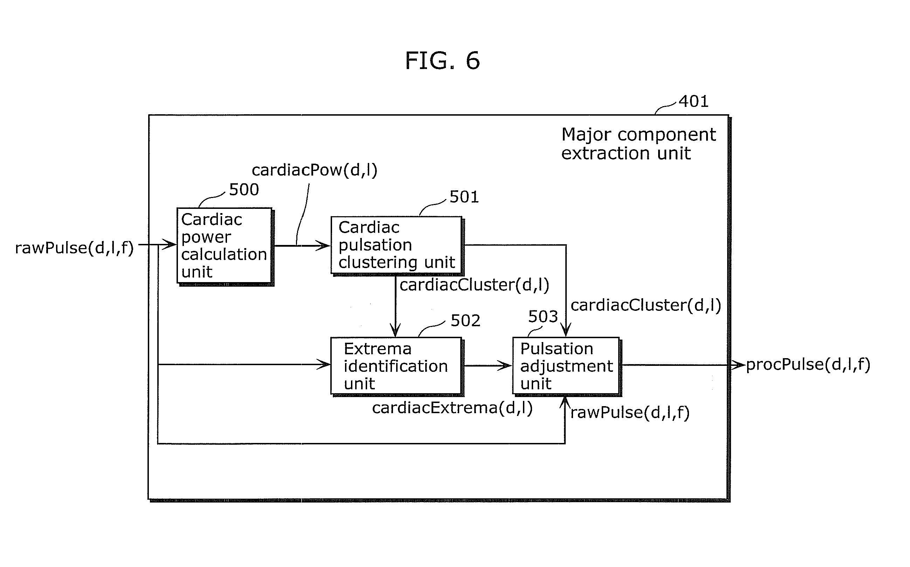

Fig. 6 is a block diagram indicating another example of the major comportment extraction unit 401. Here, it is assumed that the pulsation of interest only includes the cardiac pulsation. In other words, the major comportment extraction unit 401 extracts the cardiac pulsation from the tissue pulsation. Furthermore in Fig. 6, the major comportment extraction unit 401 includes: a cardiac power calculation unit 500, a cardiac pulsation clustering unit 501, an extrema identification unit 502, and a pulsation adjustment unit 503.

The cardiac power calculation unit 500 calculates cardiacPow(d, I) that is a signal power related to the cardiac pulsation, from the unprocessed pulsation rawPulse(d, I, f). Note that the frequency of the cardiac pulsation is assumed to be known frequency (represented by fc, usually 1 to 2 Hz for humans). In one example of implementation, cardiacPow(d, I) is calculated as a value of a power spectrum component included in an unprocessed pulsation associated with fc. In another example of implementation, cardiacPow(d, I) is calculated as a value of a spectrum component included in the unprocessed pulsation associated with fc normalized by an overall sum or partial sum of the power of all components.

The cardiac pulsation clustering unit 501 identifies a region in the tissue at which the amplitude or level of the cardiac pulsation is high, that is, cardiacCluster(d, I). In one example of implementation, cardiacCluster(d, I) is identified as a region in the tissue having cardiac power within a predetermined range. In another example of implementation, cardiacCluster(d, I) is specified to include a region having higher cardiac power in the tissue, so as to cover a predetermined percentage of the whole tissue.

The extrema identification unit 502 determines peaks and troughs (that is, extremal points) in the pulsation wave in the tissue region identified by the cardiacCluster(d, I), and outputs such peaks and troughs in the cardiac pulsation, that is, cardiacExtrema(d, I). In other words, the extrema identification unit 502 indentifies extrema of the amplitude of the cardiac pulsation in the region of the clustered tissue.

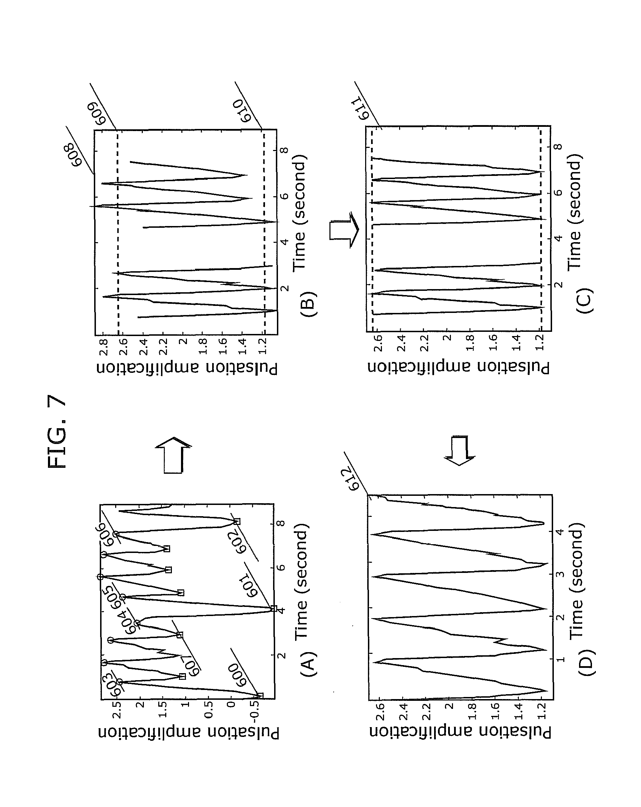

Fig. 7 shows the processing performed by the extrema identification unit 502.

An example of implementation to find the peaks and troughs is to estimate a first derivative and a second derivative of the pulsation waveform with respect to time. The peaks, which are represented by pulsePeaks(d, I), are identified at a time instance where the first derivative is zero and the second derivative is negative, and the troughs, which are represented by pulseTroughs(d, I) are identified at a time instance where the first derivative is zero and the second derivative is positive. With reference to Fig. 7(A), an example of the peaks is a point represented by points 603, 604, 605, and 606 and an example of the troughs is a point represented by points 600, 607, 601, and 602.

Since the peaks and troughs belonging to the cardiac pulsation are assumed to be more regular than those of interferences and other movements, it is possible to use, but not limited to, an outlier rejection method to execute this task. In the case of the outlier rejection, examples of peaks and troughs of the cardiac pulsation are points 603, 607, 605, and 606, and examples of peaks and troughs of interferences are points 600, 604, 601, and 602. In other words, the extrema identification unit 502 may include an extremal point identification unit (not shown) which identifies the peaks and troughs in the pulsation waveform, and an outlier rejection unit (not shown) which rejects an outlier in the amplitude of the pulsation waveform so as to eliminate an interference peak whose gap from another peak is larger than a predetermined threshold, and an interference trough whose gap from another trough is larger than a predetermined threshold.

Note that a peak of the interference (also referred to as an interference peak) is a peak larger than the other peaks by a predetermined threshold. In addition, a trough of the interference (also referred to as an interference trough) is a trough larger than the other troughs by a predetermined threshold.

The pulsation adjustment unit 503 obtains cardiacCluster(d, I) and cardiacExtrema(d, I) as information for adjusting rawPulse(d, I, f) to include only the cardiac pulsation. An output of this block is procPulse(d, I). Fig. 7 shows an advantageous effect of the processing performed by the pulsation adjustment unit 503.

In an embodiment of the pulsation adjustment unit 503, as shown by a graph 608 in Fig. 7(B), a length of time which only corresponds to the cardiac pulsation is identified from the cardiac peak and troughs. A period corresponding to the interference is eliminated from the pulsation.

In another embodiment of the pulsation adjustment unit 503, with reference to Fig. 7(C), the amplitudes of the pulsation waveform are further adjusted such that the amplitudes at each of the peaks and troughs of the cardiac pulsation are aligned (graph 611). In other words, the pulsation adjustment unit 503 adjusts the amplitudes of the pulsation waveform so that the amplitudes are aligned at each of a minimum value and a maximum value in the pulsation waveform. At this time, the peak amplitude and trough amplitude used for aligning amplitudes are, but not limited to, average amplitudes of the peaks and troughs shown by, for example, dashed lines 609 and 610 in Fig. 7(B). All the other points that are not peaks and troughs are adjusted by, but not limited to, a linear mapping method.

In another embodiment of the pulsation adjustment, fragmented cardiac durations are combined as in graphs 608 and 611. The pulsation obtained as a result is represented by a graph 612 with reference to Fig. 7(D). Such processing method ensures that an ending point of a fragmented cardiac duration must be of the same type (that is, either peak or trough) as the type of a starting point of the next fragmented cardiac duration.

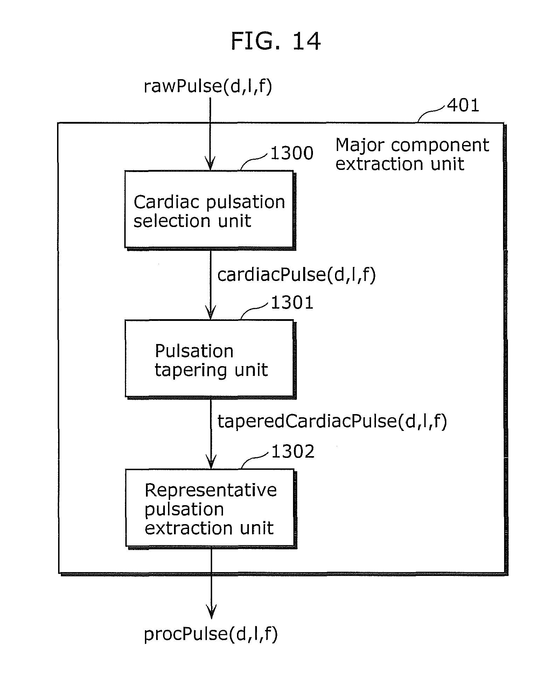

In another embodiment of the major comportment extraction unit 401, segmenting, tapering, and averaging are used to obtain a representative pulsation of the tissue block. This is shown in Fig. 14.

In another example of the embodiment of the major comportment extraction unit 401 as shown in Fig.14, the major comportment extraction unit 401 includes: a cardiac pulsation selection unit 1300, a pulsation tapering unit 1301, and a representative pulsation extraction unit 1302.

The cardiac pulsation selection unit 1300, upon receiving the pulsation rawPulse(d, I, f) as input, selects the cardiac pulsation according to a given criterion. An example of the selection criterion is a criterion such that signal energy of the cardiac frequency domain is higher than that of the other regions. Another example of the selection criterion is a criterion such that signal spectrum flatness of the cardiac frequency domain is lower than a threshold. When the cardiac pulsation is not selected, that is, when the input pulsation does not satisfy the selection criterion, the cardiac pulsation may be zero. An output of the cardiac pulsation selection unit 1300 is cardiacPulse(d, I, f).

The pulsation tapering unit 1301, upon receiving cardiacPulse(d, I, f) as input, gradually decreases respective cardiac cycles to have the same duration, so as to compare these cardiac cycles accurately. An output of this block is taperedCardiacPulse(d, I, f).

The representative pulsation extraction unit 1302, upon receiving taperedCardiacPulse(d, I, f) as input, calculates a representative pulsation at the tissue point from the tapered cardiac pulses in the pulsation waveform. An example of such calculation is to average the tapered cardiac pulses over several cycles. Another example of such calculation is calculation using PCA or ICA.

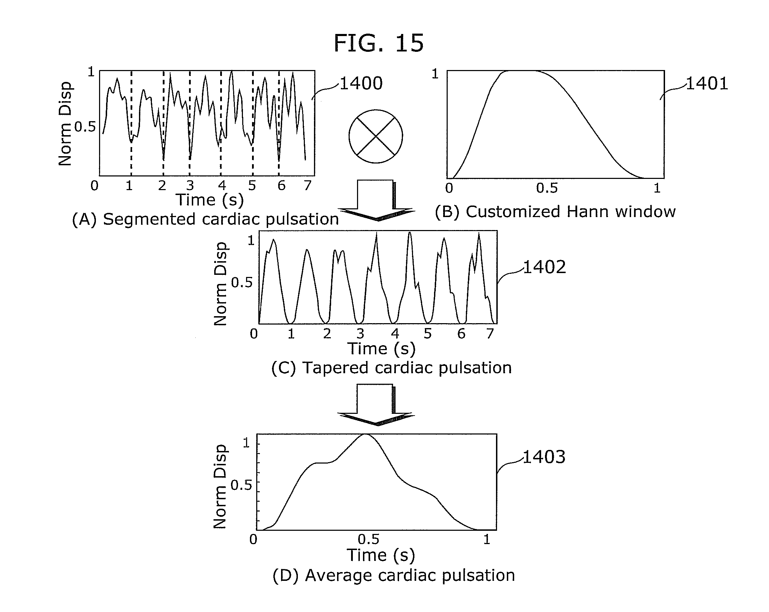

Fig. 15 shows examples of signal segmenting, tapering, and representative extraction. First, the cardiac pulses are adjusted so that all the waveform amplitudes are either positive or negative. Then, according to the starting point or ending point of the reference pulsation (reference pulsation), cardiac pulses at different tissue points are segmented into consecutive cardiac cycles. The result is shown by a graph 1400 in Fig. 15(A). An example of the reference pulsation is a strong cardiac pulse within a scan plane, and another example of the reference pulsation is an ECG signal.

After the segmentation, each cardiac cycle is gradually made smaller (tapered) to have the same duration by window function. The result is shown by a graph 1402 in Fig. 15(C). An example of this window function is a customized Hann window 1401 shown in Fig. 15(B). Note that for the window function, another arbitrary function such as a Hamming window may be used.

Lastly, the representative pulsation is obtained by averaging these tapered cardiac pulses over several cycles. The obtained representative pulsation is shown as a graph 1403 in Fig. 15(D).

The three implementations related to the major component extraction described above may be used individually or in combination, and such implementations are still within the scope of the present invention.

As output of the block segmentation unit 100, the tissue pulsation estimation unit 101, and the pre-processing unit 102, the processed pulsation may be calculated at all scan points (that is, measurement points) included in each tissue block or may be one or more representative pulsations in each tissue block. In addition, a combination of such representative pulsations may be used.

Again, with reference to Fig. 1, the pulsation-related feature extraction unit 103 extracts, upon receiving procPulse(d, l) as input, extracts pulsation-related features (that is, feature quantities) that are parameters related to the pulsation waveform in time domain. Such pulsation-related features may be all or a subset of: a pulsation amplitude feature, a pulsation shape feature, and a pulsation temporal feature. These are collectively referred to as pulseFeature.

For example, the pulsation-related feature extraction unit 103 may calculate the pulsation amplitude feature that is a feature quantity for the amplitude of the pulsation waveform. Cancerous tumors require creation of new vessels to supply nutrients and oxygen. With the increase in capillary blood supply, due to blood perfusion, the pulsation amplitude and the distribution thereof around the tissue (statistically and spatially) are likely to be higher than those of normal tissues.

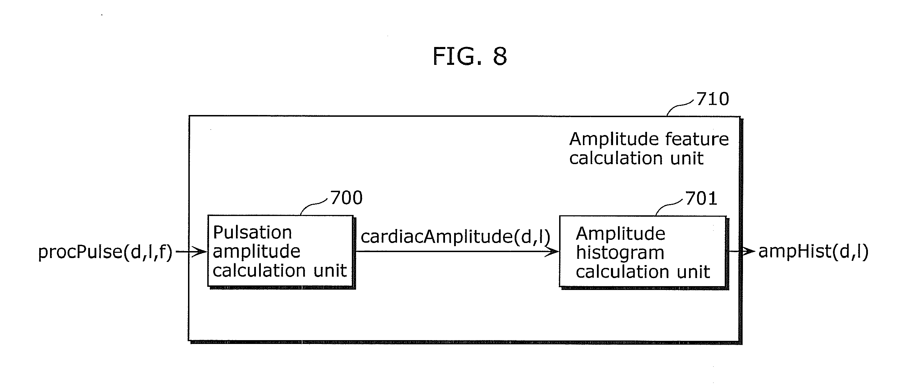

Fig. 8 shows an example of a configuration of the amplitude feature calculation unit 710 which calculates the pulsation amplitude feature.

Fig. 8 shows a functional block of the amplitude feature calculation unit 710 which calculates the pulsation amplitude feature and is included in the pulsation-related feature extraction unit 103. As shown in Fig. 8, the amplitude feature calculation unit 710 includes a pulsation amplitude calculation unit 700 and an amplitude histogram calculation unit 701.

The pulsation amplitude calculation unit 700 calculates cardiacAmplitude(d, l), which is a cardiac pulsation amplitude at each tissue point specified by d and l.

One method for calculating cardiacAmplitude(d, l) is to use, but not limited to, a difference between an average peak amplitude and an average trough amplitude of the pulsation.

In one embodiment of the amplitude histogram calculation unit 701, the calculated cardiacAmplitude(d, l) in all the tissue points or tissue blocks is grouped into various predetermined bins. The amplitude histogram calculation unit 701 outputs "1" when the tissue point or tissue block is where the pulsation amplitudes belong to the bins, and outputs "0" otherwise. The amplitude in each bin can be set individually, and the range of one bin does not affect the range of another bin. Both values of cardiacAmplitude(d, l) and the clustering result can be used as output of the amplitude feature calculation unit 710.

An example of such a bin is [0.5, 1], which hypothetically corresponds to the tumoral pulsation amplitude in terms of tissue skewness. Another example is [0.1, 0.5], which hypothetically corresponds to the pulsation amplitude of a normal tissue in terms of tissue skewness. The other bins may be defined based on a specific hypothesis regarding the pulsation amplitude, and such implementations are still within the scope of the present invention.

In another embodiment of the amplitude histogram calculation unit 701, the amplitude histogram calculation unit 701 may segment the whole scanned tissue into subregions, calculate an amplitude histogram for each subregion, and output a subregion in which the histogram follows a predetermined pattern. An example of tissue segmentation is a rectangular grid. An example of an amplitude histogram is probability distribution of the pulsation amplitude over an entire set of predetermined bins. The amplitude in each histogram bin can be set individually, and the range of one bin does not affect the range of another bin. An example of a predetermined pattern is such that the probability distribution is biased toward the range [0.5, 1].

Note that as pulseAmpFeatures that is output of the amplitude feature calculation unit 710, either an absolute value cardiacAmplitude(d, l) of the pulsation or a clustering result ampHist(d, l) based on the histogram may be selected.

In addition, the pulsation-related feature extraction unit 103 may calculate the pulsation shape feature that is a feature quantity regarding the shape of the tissue pulsation waveform. New vessels associated with cancerous tissues have perivascular detachment, vessel dilation, and irregular shape. Tumoral blood vessels do not have smooth muscle cells which exist in the blood vessels in normal tissues and are not formed sufficiently to provide oxygen to all the cancerous tissues. Furthermore, tumoral blood vessels are more porous and leaky than blood vessels in normal tissues. These factors cause the tumoral pulsation to have a more dilated shape than the pulsation of normal tissues.

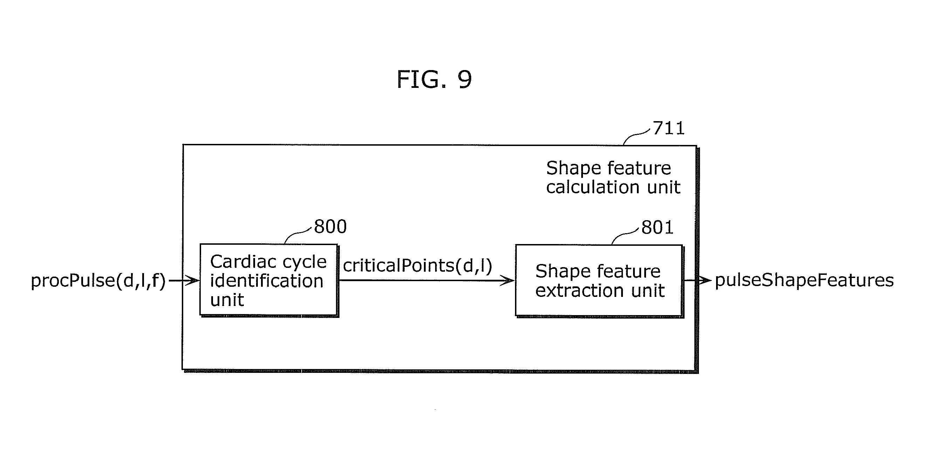

Fig. 9 shows a functional block of a shape feature calculation unit 711 which calculates the pulsation shape feature and is included in the pulsation-related feature extraction unit 103. As shown in Fig. 9, the shape feature calculation unit 711 includes a cardiac cycle identification unit 800 and a shape feature extraction unit 801.

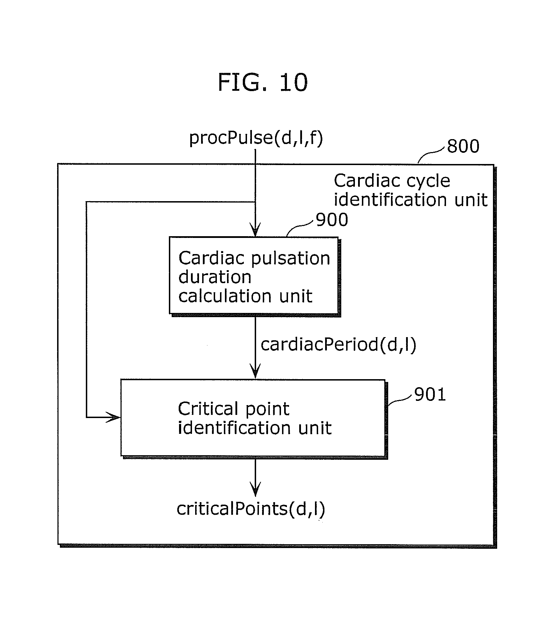

The cardiac cycle identification unit 800 identifies each cardiac cycle from procPulse(d, l, f). Critical points of each cardiac cycle, that is, criticalPoints(d, l), can be extracted from this block. Fig. 10 shows one implementation of a cardiac cycle identification method. The cardiac cycle identification unit 800 in this implementation includes a cardiac pulsation duration calculation unit 900 and a critical point identification unit 901.

The cardiac pulsation duration calculation unit 900 calculates an average cardiac pulsation duration of an input pulsation from procPulse(d, l, f). Note that the cardiac pulsation duration calculation unit 900 may calculate the cardiac cycle of each input pulsation.

An example of this calculation is to transform the input pulsation into frequency domain, search the fundamental frequency component, and select the searched component as the average cardiac pulsation duration.

Note that another example of this calculation may be such that the pulsation adjustment unit 503 calculates an average distance between two adjacent peaks or two adjacent troughs, and selects the average distance as the average cardiac cycle (that is, the average cardiac pulsation duration).

An output of the cardiac pulsation duration calculation unit 900 is cardiacPeriod(d, l), where d represents depth and l represents line.

The critical point identification unit 901 identifies the critical points of the cardiac cycle at each measurement point. Critical points, for example, can be considered to include, but not limited to, a systolic starting point, a diastolic ending point, a systolic peak, a systolic mid-point, and a diastolic mid-point. The input to this block includes procPulse (d, l, f) and cardiacPeriod(d, l), and the output from this block is criticalPoints(d, l). Here, d represents depth, and l represents line.

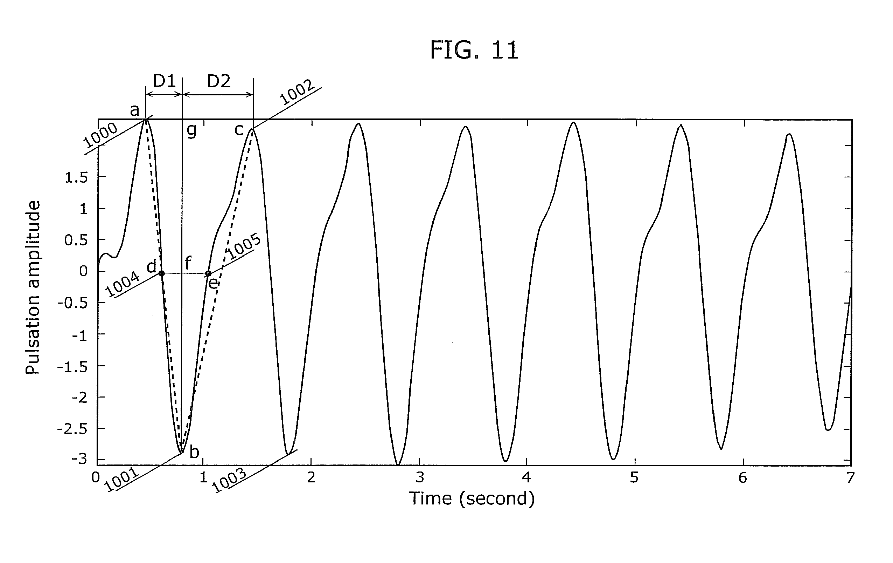

An example of processing performed by the critical point identification unit 901 is described with reference to Fig. 11.

A first step in the processing performed by the critical point identification unit 901 is to search a maximum point and a minimum point at procPulse(d, l). An example of this step has the following procedure: (1) a first maximum point 1000 is searched within a cardiac cycle from time 0 second, (2) a first minimum point 1001 is searched within a cardiac cycle from time corresponding to the first maximum point 1000, (3) a second maximum point 1002 is searched within a cardiac cycle from the first minimum point 1001, and (4) the processing in (1) to (3) described above is repeated until a desired number of maximum and minimum points are searched.

A second step performed by the cardiac pulsation duration calculation unit 900 is to determine a pulsation direction. There can be two types of pulsation direction, which is: an upward direction (having an upward systolic curve and then a downward diastolic curve), or a downward direction (having a downward systolic curve and then an upward diastolic curve) of one entire cardiac cycle. It is assumed that a systolic part of the cardiac pulsation has a shorter duration than that of a diastolic part. Note that the systolic curve is a pulsation waveform in the systolic duration of the heart, and the diastolic curve is a pulsation waveform in the diastolic duration of the heart.

An example of a method for determining the pulsation direction is to compare the gradients of lines ab and bc shown in Fig. 11. Points a and c are two consecutive local maximums, and a point b is a local minimum between the points a and c. The gradients of the lines ab and ac may be an average of a predetermined number of cardiac cycles. If the gradient of the line ab is larger than the gradient of the line bc, the pulsation direction is downward, and the point a is the starting point of this cardiac cycle. If the gradient is smaller, the pulsation direction is upward, and the point b is the starting point of one cardiac cycle.

Another example of the method for determining the pulsation direction is to compare lengths of D1 and D2. D1 is a period from the first maximum point 1000 to the first minimum point 1001 adjacent to the first maximum point 1000. D2 is a period from the first minimum point 1001 to the second maximum point 1002. D1 and D2 may be an average of a predetermined number of cardiac cycles. The pulsation direction is downward when D2 is larger than D1, and the pulsation direction is upward when D2 is smaller.

The last step of the processing performed by the critical point identification unit 901 is to identify the critical points in each cardiac cycle. Critical points include, but not limited to, the systolic starting point, the diastolic ending point, the systolic peak, the systolic mid-point, and the diastolic mid-point. The systolic mid-point and the diastolic mid-point are two points in the systolic curve and the diastolic curve when the pulsation amplitude is equal to a predetermined ratio with respect to a maximum pulsation amplitude. An example of this ratio is half the maximum pulsation amplitude. Assuming that the pulsation direction is downward in Fig. 11, the systolic starting point is the first maximum point 1000. The diastolic ending point is the next second maximum point 1002. The systolic peak is the first minimum point 1001. The systolic mid-point 1004 and the diastolic mid-point 1005 are two points d and e located on the systolic curve and the diastolic curve and corresponding to an amplitude f at which bf/bg equals a predetermined value.

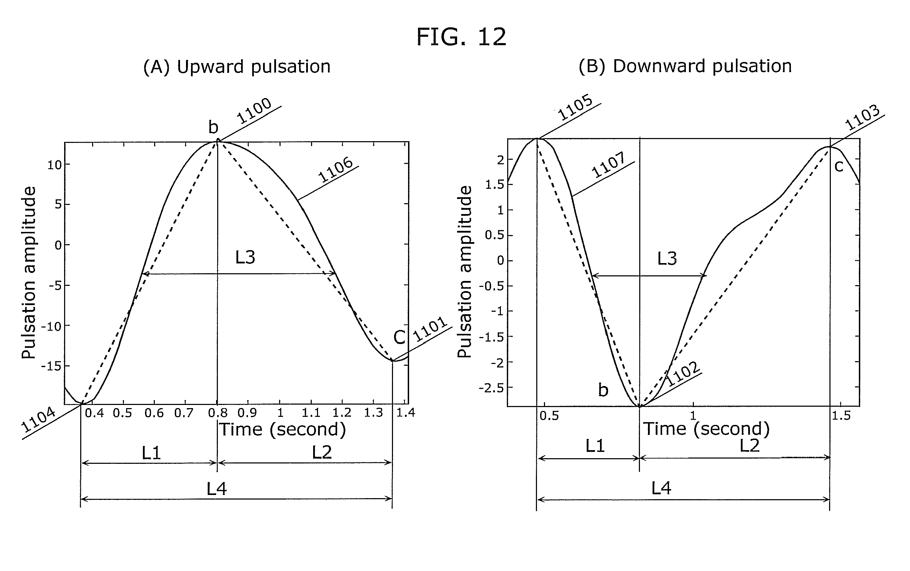

The shape feature extraction unit 801 extracts a useful feature related to the pulsation waveform shape. This feature is described with reference to Fig. 12.

The feature related to the pulsation waveform shape includes, but not limited to, L1/L2, L3/L4, pulsation skewness, pulsation kurtosis, a deviation of the diastolic curve bc with respect to a straight line bc, and a deviation of extrema in the diastolic curve bc. Note that the straight line bc may be a predetermined curve.

L2(d, l) is a duration of the diastolic part of the cardiac cycle. More specifically, in the case of the upward cardiac cycle, with reference to Fig. 12(A), L2(d, l) is a period from the maximum point 1100 to the diastolic ending point 1101, and in the case of the downward cardiac cycle, with reference to Fig. 12(B), it is a period from the minimum point 1102 to the diastolic ending point 1103.

L1(d, l) is a duration of the systolic part of the cardiac cycle. More specifically, in the case of the upward cardiac cycle, with reference to Fig. 12(A), L1(d, l) is a period from the systolic starting point 1104 to the highest point 1100, and in the case of the downward cardiac cycle, with reference to Fig. 12(B), it is a period from the systolic starting point 1105 to the maximum point 1102.

Due to the irregular structure of the microvascular system in cancerous tissues, the tumoral pulsation is likely to have a delay in the systolic peak or have a longer systolic duration. Higher L1/L2 indicates a higher systolic duration. This means that the corresponding pulsation has higher probability of being tumoral pulsation.

L3(d, l) is defined as an amplitude when the pulsation amplitude equals the predetermined ratio of the maximum amplitude, that is, as the amplitude during a period from the systolic mid-point to the diastolic mid-point. Note that systolic mid-point is a point which is located on the systolic curve and at which the amplitude of the systolic curve, in the systolic duration, equals a ratio predetermined with respect to the maximum amplitude. In addition, the diastolic mid-point is a point which is located on the diastolic curve and at which the amplitude of the diastolic curve, in the diastolic duration, equals the ratio predetermined with respect to the maximum amplitude.

L4(d, l) is one pulse duration. The ratio L3/L4 is used to measure sharpness of the pulse. A tumoral pulsation is likely to have a wider, that is, a more dilated shape than that of a normal pulsation. Higher L3/L4 indicates a more dilated pulsation shape. This indicates that the corresponding pulsation has higher probability of being tumoral pulsation.

Skewness is a measure of asymmetry of the cardiac pulsation, and is represented by pulseskewness(d, l). An example of the method for calculating pulseskewness(d, l) is to use Expression 1 below.

[Math. 1]

$$pulseSkewness(d,l) = \frac{1}{N}\sum_{f=1}^{N}(\frac{procPulse(d,l,f)-\mu}{\sigma})^3 \qquad \text{(Expression 1)}$$

Here, N is the number of frames in a cardiac cycle, and $\mu$ and $\sigma$ are a mean and a standard deviation of the pulsation amplitudes within a certain cardiac cycle. As with L1/L2, if the systolic duration is shorter and the diastolic duration is longer, the pulsation has higher probability of being normal pulsation. A more positive kurtosis indicates that the diastolic duration of the cardiac cycle is longer than the systolic duration of the cardiac cycle, which indicates a smaller possibility of being tumoral pulsation.

A positive kurtosis is a measure of sharpness of the systolic peak with respect to the normal distribution, and is represented by pulseKurtosis(d, l). An example of the method for calculating pulseKurtosis(d, l) is to use Expression 2 below.

[Math. 2]

$$pulseKurtosis(d,l) = \left[\frac{1}{N}\sum_{f=1}^{N}\left(\frac{procPulse(d,l,f)-\mu}{\sigma}\right)^4\right] - 3 \qquad \text{(Expression 2)}$$

As with L3/L4, a narrower, that is, sharper pulsation has higher probability of being normal pulsation. A more positive kurtosis indicates that a cardiac pulsation part near the systolic peak is sharper, which indicates a lower probability of being tumoral pulsation.

With reference to Fig. 12, a deviation of the diastolic curve bc with respect to the straight line bc is calculated as a sum of differences between an amplitude of the diastolic curve bc and an amplitude of the straight line bc. This is calculated by Expression 3 below in consideration of the cardiac pulsation direction denoted as pulseCurveDeviation(d, l).

[Math. 3]

$$pulseCurveDeviation(d,l) = \begin{cases} \sum_{bc}\big(procPulse(d,l,f) - straightLine_{bc}(d,l,f)\big) \text{ if cardiac direction is upward} \\ \sum_{bc}\big(straightLine_{bc}(d,l,f) - procPulse(d,l,f)\big) \text{ if cardiac direction is downward} \end{cases}$$

(Expression 3)

The result is used to measure to what extent the curve is curved. Positive pulseCurveDeviation(d, l) means that the pulsation curve is more dilated, and negative pulseCurveDeviation(d, l) means the opposite. This calculation proves that the pulsation curve 1106 is more dilated when it has positive pulseCurve Deviation(d, l), and a pulsation curve 1107 is less dilated when it has negative pulseCurveDeviation(d, l). A more dilated pulsation indicates a higher probability of being tumoral pulsation.

A deviation of the extrema in the diastolic curve bc is calculated as a difference in pulsation amplitude between the maximum value and the minimum value within the diastolic curve bc, and is denoted as pulseExtremaDeviation(d, l). When the diastolic curve bc has no extrema, the deviation of the extrema is zero. Generally, due to the vasodilation state of the microvascular system in cancerous tissues, no dicrotic notch exists in the pulsation. Thus, higher pulseExtremaDeviation(d, l) indicates higher probability of existence of the dicrotic notch and lower probability of being tumoral pulsation.

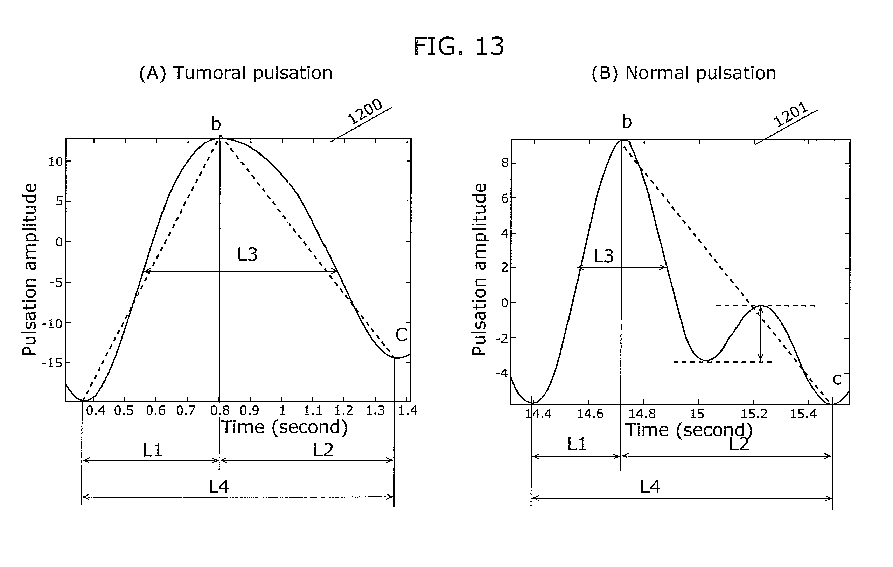

Fig. 13 shows an example of a comparison between the features of the tumoral pulsation and the normal tissue pulsation.

As shown in Fig. 13(A), higher L1/L2, higher L3/L4, lower skewness, lower kurtosis, a more positive deviation of the pulsation curve, and a lower deviation of the extrema in the diastolic curve mean that the waveform is more dilated (graph 1200), and indicate higher probability of being a tumoral pulsation waveform. Whereas, as shown in Fig .13(B), lower L1/L2, lower L3/L4, higher skewness, higher kurtosis, a more negative deviation of the pulsation curve, and a higher deviation of the extrema in the diastolic curve mean that the waveform is less dilated (graph 1201), and indicate lower probability of being a tumoral pulsation waveform.

In another implementation of the shape feature extraction unit 801, the value of any one of the feature quantities (for example, at least one of L1/L2, L3/L4, pulseskewness(d, l), pulseKurtosis(d, l), pulseCurveDeviation(d, l), and pulseExtremaDeviation(d, l)) may be normalized by a predetermined value (for example, a value obtained from the region known as normal tissues), or may be normalized within a predetermined range (for example, [0, 1]).

In yet another implementation of the shape feature extraction unit 801, a value of an arbitrary feature quantity or a value obtained by normalizing the value may be reversed such that a higher value indicates higher probability of being a tumoral pulsation and a lower value indicates lower probability of being a tumoral pulsation. A more specific reciprocal may be calculated. This applies to pulseskewness(d, l), pulseKurtosis(d, l), and pulseExtremaDeviation(d, l).

An original value of the pulsation-related feature (that is, the value of the pulsation-related feature before normalized), a normalized value of the pulsation-related feature, and a value corresponding to the reciprocal of the pulsation-related feature are collectively referred to as "feature values".

The feature values are used to segment the tissue in the scan area according to high or low probability of being a tumoral pulsation. An implementation of this segmentation is, but not limited to, setting a threshold for each feature and classify the regions having high and low probabilities of being tumoral pulsations, based on these thresholds. An example of threshold selection is to use a mean of the feature values.

The output pulseShapeFeatures of the shape feature extraction unit 801 can be selected as feature values or a clustering result based on the feature values.

With reference to Fig. 1 again, the pulsation-related feature extraction unit 103 may further calculate a pulsation temporal feature that is a feature quantity with respect to a temporal axis of the tissue pulsation waveform. Differences in pulsation amplitude and shape lead to a difference of the entire pulsation waveform of the cancerous tumor from that of the normal tissue. Furthermore, a difference in resistance to blood flow due to the microvascular structure leads to the difference in pulsation arrival time between the cancerous tissue and the normal tissue.

This pulsation temporal feature is, for example, at least one of: a cardiac cycle delay, a cardiac waveform difference, and an autoregressive coefficient of the cardiac waveform. These are collectively referred to as pulseTemporalFeatures.

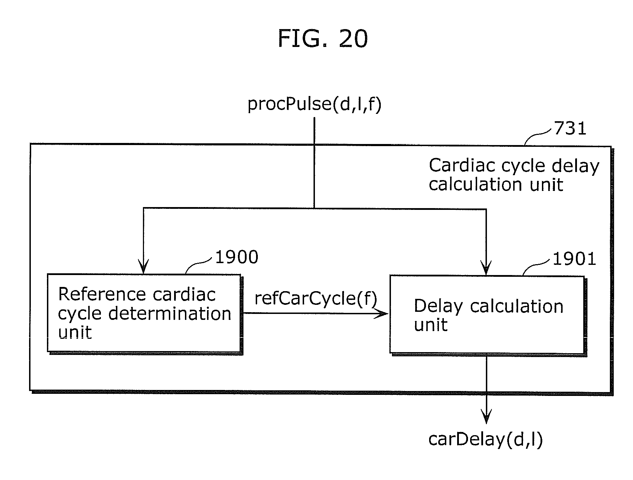

Fig. 20 is an example of a functional block of a cardiac cycle delay calculation unit 731 which calculates a cardiac cycle delay amount and is included in the pulsation-related feature extraction unit 103. As shown in Fig. 20, the cardiac cycle delay calculation unit 731 includes a reference cardiac cycle determination unit 1900 and a delay calculation unit 1901.

An input to the cardiac cycle delay calculation unit is pulsation, that is, procPulse(d, l, f). Then, the output is a reference pulsation (or reference cardiac cycle), which is a delay of each pulsation (made up of cardiac cycles) with respect to carDelay(d, l). The cardiac cycle delay means resistance of blood vessels to blood flow.

The reference cardiac cycle determination unit 1900 detects the reference pulsation or reference point of the cardiac cycle with which all the pulsations are compared. An example of such reference is an electrocardiography (ECG) signal. This represents the exact starting point and systolic peak of the cardiac cycle. When the ECG signal is not available, another example of such reference is a strongest pulsation in the scanned tissue. An output of the reference cardiac cycle determination unit 1900 is refCarCycle(f).

The delay calculation unit 1901 calculates the starting point of the cardiac cycle or the delay of the systolic peak in procPulse(d, l, f) with respect to the reference refCarCycle(f). Note that resampling to standardize the cardiac cycle length may be performed before the calculation, so as to allow comparison with the refCarCycle(f).

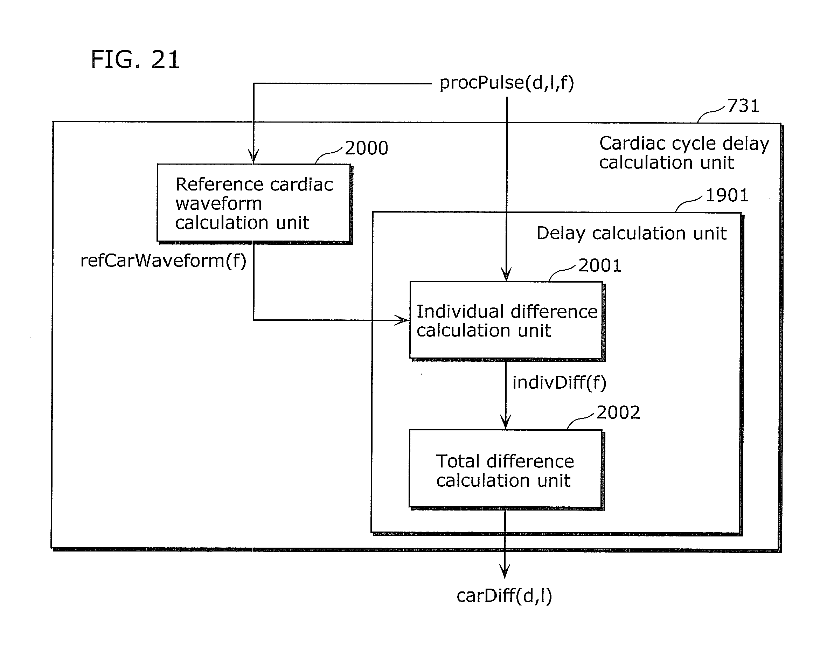

Fig. 21 shows an example of a functional block of the delay calculation unit 1901 which calculates the cardiac waveform difference. An input to the delay calculation unit 1901 is the pulsation procPulse(d, l, f) and carDelay(d, l), and an output is a cardiac cycle difference in pulsation, that is, carDiff(d, l). Such a value determines to what extent the cardiac cycles are similar to or different from each other.

As shown in Fig. 21, the delay calculation unit 1901 includes an individual difference calculation unit 2001 and a total difference calculation unit 2002.

Note that the reference cardiac waveform calculation unit 2000 calculates the reference cardiac waveform with which all the obtained cardiac waveforms are compared. In one embodiment, such a reference cardiac waveform is calculated as an average of all the obtained cardiac waveforms. In another embodiment, such a reference cardiac waveform is calculated as the strongest cardiac waveform of all the obtained cardiac waveforms. In yet another embodiment, such a reference cardiac waveform may be defined without considering the obtained cardiac waveform, for example, a known cardiac waveform taken from literature. An output of this block is refCarWaveform(f).

For the cardiac waveform of each pulsation procPulse(d, l, f), the individual difference calculation unit 2001 calculates the difference between the cardiac waveform and the reference cardiac waveform refCarWaveform(f). Prior to this calculation, these cardiac waveforms may be gradually tapered to have the same length. An example of the difference calculated by the individual difference calculation unit 2001 is a sum of absolute differences between each pulsation procPulse(d, l, f) and refCarWaveform(f). Another example of such a difference is a root mean square difference.

The total difference calculation unit 2002 determines one overall difference value per pulsation, from all the individual difference values calculated by the individual difference calculation unit 2001. An example of such an overall difference value is a standard deviation of the individual difference values.

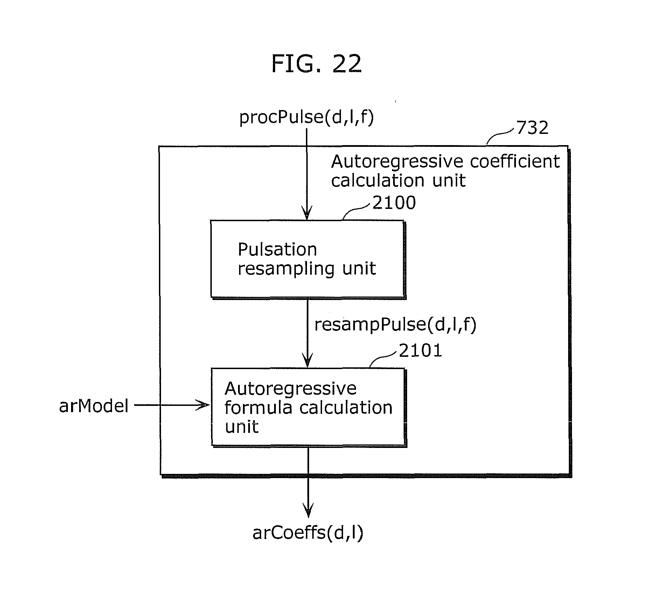

Fig. 22 is an example of a functional block of an autoregressive coefficient calculation unit 732 which calculates an autoregressive coefficient of the waveform representing the cardiac pulsation and is included in the pulsation-related feature extraction unit 103. As shown in Fig. 22, the autoregressive coefficient calculation unit 732 includes a pulsation resampling unit 2100 and an autoregressive formula calculation unit 2101.

Generally, autoregressive coefficients are used to describe any waveform as an autoregressive process. In other words, the current sample is a linear combination of past samples. Thus, these autoregressive coefficients may be used to describe the pulsation. The input to the autoregressive coefficient calculation unit 732 is the pulsation procPulse(d, l, f), and the output is a model coefficient, that is, arCoeffs(d, l).

The pulsation resampling unit 2100 resamples pulsations so that each cardiac cycle contains a standardized number of samples. For example, in the case of a frame rate of 40 frames per second (that is, the sampling rate in time domain is 40 Hz) and a heart rate of one beat per second, one cardiac cycle contains 40 samples. However, this number may vary, for example, when the ultrasound transducer has a different configuration and physiological conditions are different. When the number is different from 40 samples, the pulsations are resampled so that one cardiac cycle contains 40 samples. An output from this block is resampPulse(d, l, f).

The autoregressive formula calculation unit 2101 finds a solution (that is, the autoregressive coefficient) of an autoregressive formula. This is applied to the pulsation.

Generally, the autoregressive formula is described as Expression 4 below.

[Math. 4]

$$X_t = \sum_{i=1}^{p} \varphi_i X_{t-i} + \varepsilon_t$$

(Expression 4)

Here, Xt is the current sample, Xt-i is the past sample, p is a model order, φi is a model coefficient, and εt is white noise. According to an experiment by the inventors, when each cardiac cycle includes 40 samples, an order of 5 is sufficient to describe the pulsation with a smaller error (that is, a root mean square error of less than 1 %).

This expression is applied to the obtained pulsation. In literature, a solution for such a problem has been proposed, and one example is Yule-Walker equations. Since this is not within the scope of the present invention, the description thereof is omitted.

The solution of the model coefficient is the output feature, that is, arCoeffs(d, l).

All the features described above (carDelay(d, l), carDiff(d, l), and arCoeffs(d, l)) are collectively referred to as pulseTemporalFeatures.

The pulsation-related feature extraction unit 103 may output, as output thereof, all or a subset of pulseAmp-Featuers, pulseShapeFeatures, and pulseTemporalFeatures.

With reference to Fig. 1 again, after calculating the pulsation-related features, the distribution characteristics calculation unit 104 in Fig. 1 calculates distribution characteristics of these features in each tissue block or across a plurality of tissue blocks.

The irregular shape of the microvascular system in cancerous tumors can be quantified by such distribution characteristics calculated from B-mode grayscales or pulsation-related features. In an application example where the B-mode resolution is not high enough to visualize groups of microvessels (in most clinical applications, the resolution is not sufficiently high for this purpose), it is more preferable to calculate the distribution characteristics from the calculated pulsation-related features derived from microvascular pulsations. The output is a distribution feature, that is, distributionFeature.

For the distribution feature, it is possible to consider using at least one of a statistical distribution parameter and a spatial distribution parameter.

The statistical distribution parameter is, for example, a mean of the feature values (that is, pulsation-related features); a median of the feature values; a largest feature value; a smallest feature value; a standard deviation of the feature values; kurtosis of the feature values; and skewness of the feature values.

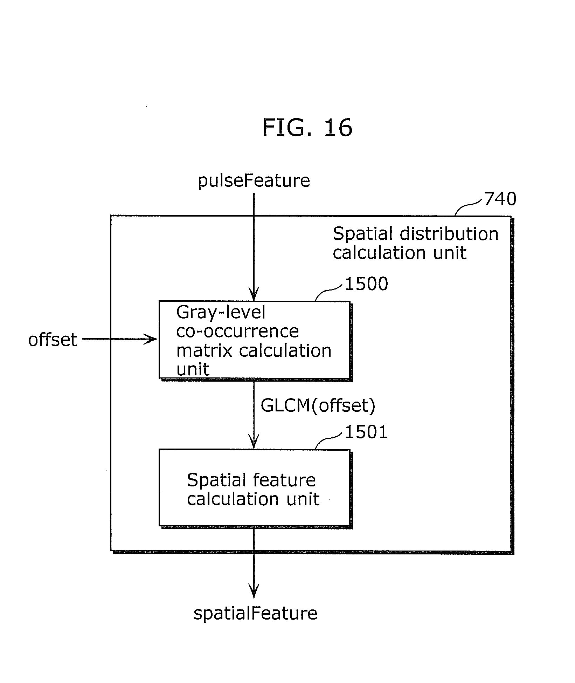

The spatial distribution parameter is, for example, at least one of: energy of the feature values; entropy of the feature values; contrast of the feature values; homogeneity of the feature values; and correlation of the features values. Fig. 16 shows the calculation performed on all of these parameters.

Fig. 16 shows a functional block of the spatial distribution calculation unit 740 which calculates the spatial distribution parameter and is included in the distribution characteristics calculation unit 10.

As shown in Fig. 16, the spatial distribution calculation unit 740 includes a gray-level co-occurrence matrix calculation unit 1500 and a spatial feature calculation unit 1501.

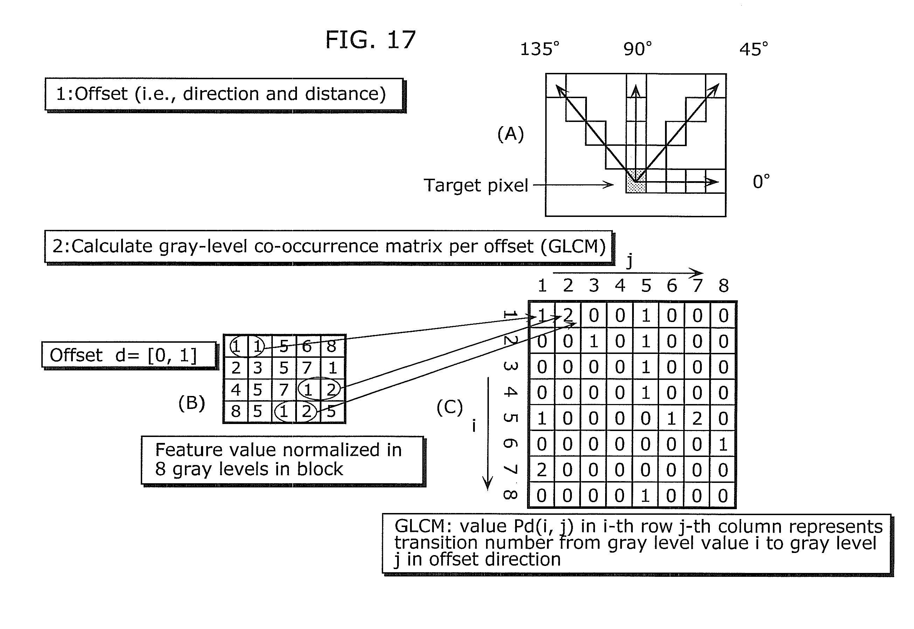

The gray-level co-occurrence matrix calculation unit 1500 calculates how often a feature of a value i occurs in a specific spatial relationship with a feature of a value j in a block. Such a specific spatial relationship represents various directions and distances, and is referred to as an offset. As an example of the offset, Fig. 17 shows four specific directions (horizontal, vertical, and two diagonal directions) and distances for each direction.

The spatial feature calculation unit 1501 calculates an average statistical feature from the co-occurrence matrix. This feature includes, but not limited to, at least one of the following:

[0216] - Energy (assuming as the sum of squared elements in the co-occurrence matrix, also known as uniformity or angular second moment). This is a measure of textual uniformity, that is, whether or not the feature value is spatially repeated. A larger value indicates more uniformity of the feature (that is, there is a repetitive structure).

[0217] - Contrast (to measure local variation in the co-occurrence matrix). This is a measure of local variation. Contrast is higher when the feature values show large and abrupt changes in space. In this case, homogeneity is lower.

[0218] - Homogeneity (on-diagonal element of the co-occurrence matrix). Homogeneity is higher when the range of feature values is small. In this case, homogeneity is higher.

[0219] - Correlation (to measure joint probability at which a specific feature pair occurs). Correlation is higher when the feature values are spatially closer to a linear structure.

[0220] - Entropy (a measure of randomness of the features). This is a measure of homogeneity of the co-occurrence matrix of features. Entropy is maximum when the matrix elements are equal, which indicates that the feature value has a larger degree of change.

Formulae for calculating a spatial feature as described above are represented as Expressions 5 to 9 below.
[Math. 5]

$$Energy = \sum_i \sum_j P_d^2(i,j)$$

(Expression 5)

[Math. 6]

$$Entropy = -\sum_i \sum_j P_d(i,j) \log P_d(i,j)$$

(Expression 6)

[Math. 7]

$$Contrast = \sum_i \sum_j (i-j)^2 P_d(i,j)$$

(Expression 7)

[Math. 8]

$$Homogeneity = \sum_i \sum_j \frac{P_d(i,j)}{1+|i-j|}$$

(Expression 8)

[Math. 9]

$$Correlation = \frac{\sum_i \sum_j (i-\mu_x)(j-\mu_y) P_d(i,j)}{\sigma_x \sigma_y}$$

(Expression 9)

Here, $P_d$ is a gray-level co-occurrence matrix, and an entry (i, j) of $P_d$ is the number of occurrences of a pair of gray levels i and j that are apart at a distance d. $\sigma_x$ and $\sigma_y$ are standard deviations of $P_d(x)$ and $P_d(y)$, respectively, and $\mu_x$ and $\mu_y$ are means of $P_d(x)$ and $P_d(y)$, respectively. Note that $P_d(x)$ and $P_d(y)$ are represented by Expression 10 and Expression 11 below, respectively.
[Math. 10]

$$P_d(x) = \sum_j P_d(x, j)$$

(Expression 10)

[Math. 11]

$$P_d(y) = \sum_i P_d(i, y)$$

(Expression 11)

Fig. 17 shows an example of calculation processing of the gray-level co-occurrence matrix (also referred to as GLMC) performed by the gray-level co-occurrence matrix calculation unit 1500.

Here, all the feature values are normalized to certain gray levels and are considered to be an input image shown in Fig. 17(B). Due to the factor that the offset is [0 1] (one distance in the right direction) as shown in Fig. 17(A), an element (1, 1) includes a value 1 in the output of the GLCM shown in Fig. 17(C). This is because in the input image shown in Fig. 17(B), there is only one instance where both of two horizontally adjacent pixels have a value of 1. In addition, in the output of the GLCM shown in Fig. 17(C), an element (1, 2) includes a value of 2. This is because in the input image shown in Fig. 17(B), there are two instances where two horizontally adjacent pixels have values of 1 and 2. Furthermore, in the output of the GLCM shown in Fig. 17(C), an element (1, 3) includes a value of 0. This is because in the input image shown in Fig. 17(B), there is no instance where two horizontally adjacent pixels have values of 1 and 3. Likewise, the gray-level co-occurrence matrix calculation unit 1500 continues processing the input image, scans the input image with respect to anther pixel pair (i, j), and records the sum in a corresponding element of the GLCM.

Next, feature ranking and feature selection performed prior to the classification by the malignancy classification unit 105 will be described. This processing is optional, and implementations that do not include this block are still within the scope of the present invention.

The number of features calculated using the methods described herein ranges from less than one hundred to several hundreds, depending on the application example and the specific implementation. In many application examples, such a large number of features might not be preferable when real-time operation is required.

A feature ranking algorithm may be used to rank the features, which allows selecting a top feature for the subsequent classification task. A criterion for feature ranking may be a class-separability criterion which describes how well the features are separated between a cancerous data group and a normal data group. The better the feature is separated, the higher the feature is ranked. Another feature ranking method may be intuitively grouping the features using a classification algorithm for evaluating performance, so as to select the best configuration.

According to an animal experiment conducted by the inventors, 10 to 50 top rank features are sufficient to improve a tradeoff relationship between classification accuracy and calculation efficiency. Thus, it is necessary to include top-ranked features in the classification task.

According to a unique experiment by the inventors, such top features are as follows:

**[0237]** - Median, entropy, standard deviation, mean, and largest value of the pulsation amplitudes in each block, at a plurality of measurement points included in each block.

**[0238]** - Median, entropy, standard deviation, mean, and largest value of the cardiac waveform difference in each block, at the measurement points included in each block.

**[0239]** - Median of L1/L2 in each block, at the measurement points in each block.

**[0240]** - L1/L2 from a representative pulsation in each block.

**[0241]** - A largest value and standard deviation of the diastolic deviation in each block, at the measurement points included in the block.

As will be understood, these features are unique to the animal experiment conducted by the inventors.

The malignancy classification unit 105 in Fig. 1 combines the outputs from the pulsation-related feature extraction unit 103 and the distribution characteristics calculation unit 104 to calculate malignancy information of the scanned tissue, that is, maligScores. This processing is performed using the classification algorithm.

An example of the classification algorithm is, but not limited to, an AdaBoost and a support vector machine. Prior to the malignancy classification, the features may be ranked and selected. Such implementations are also within the scope of the present invention.

The classification algorithm, upon receiving the calculated features as input for each block (pulseFeature, distributionFeature), outputs a malignancy score indicating whether the block is normal or malignant under a predetermined setting. As an example of the setting predetermined depending on the classification algorithm to be actually used, any of the following may be selected: the features used for the classification; the parameters used for the algorithm to calculate an intermediate output value from the feature value; and a threshold used for the algorithm to determine the malignancy score from the intermediate output value. Such a predetermined setting is obtained through the training process by which an experiment of collecting real data having known characteristics (that is, information about normal and malignant tissues is known) is conducted, and the classification algorithm is trained using the data and the characteristics. The predetermined setting can be determined by, for example, a learning algorithm based on, as teaching data, feature quantities that are previously labeled with at least one of the malignant tumor, the benign tumor, and the normal tissue. The details thereof are not within the scope of the present invention, and are not therefore described herein. Note that the malignancy classification unit 105 need not necessarily use the learning algorithm, and may classify, using a quartile point, for example, a block having an outlier as the malignant block, or may classify, as the malignant block, a block whose difference from a descriptive statistic such as a mean or median is more than or equal to a threshold or less than a threshold.

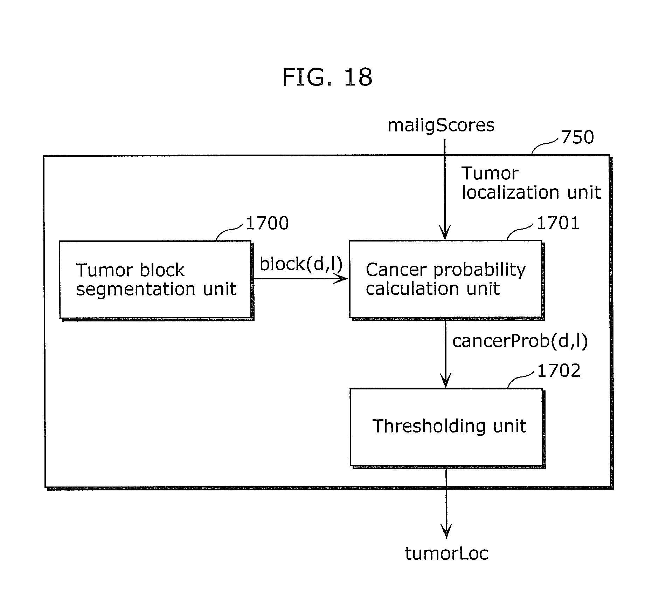

In another embodiment of the malignancy classification unit 105, the result of the classification may be further processed, to obtain a cancer probability of the scanned region and locate the position of the cancerous tumor. Fig. 18 shows an example of a functional block of the tumor localization unit which locates the position of the tumor and is included in the malignancy classification unit 105.

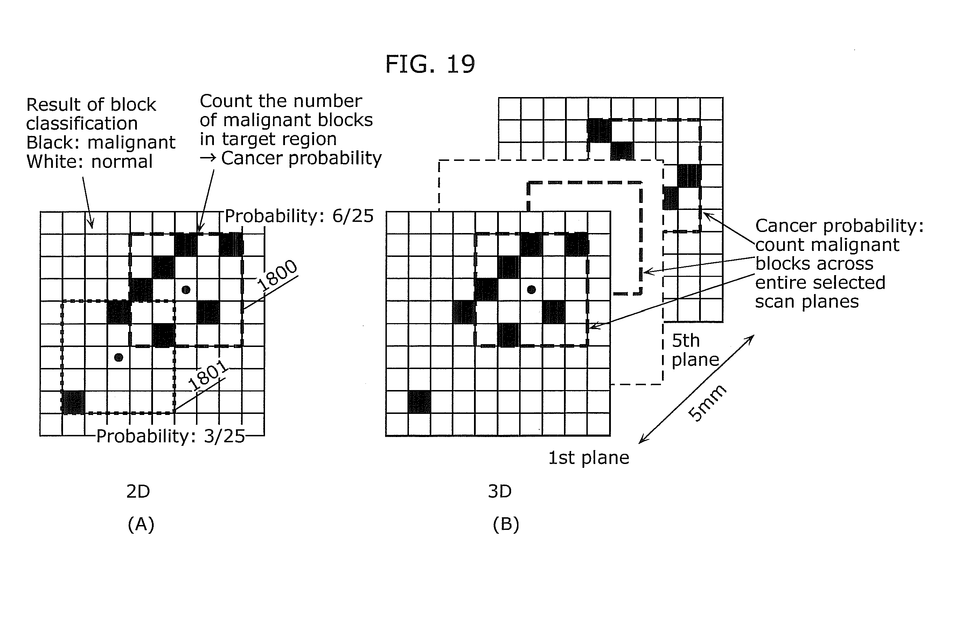

As shown in Fig. 18, the tumor localization unit 750 includes a tumor block division unit 1700, a cancer probability calculation unit 1701, and a thresholding unit 1702. Processing performed by these units is further shown in Fig. 19.

The tumor block division unit 1700 defines a target region for calculating the cancer probability for each tissue point in the scanned tissue, to find the tissue block belonging to the target region. For example, in the case of two dimensions, the shape of the target region may be square, and the size thereof may be 5 millimeters × 5 millimeters (see target areas 1800 and 1801 in Fig. 19(A)). In the case of three dimensions, the shape of the target region may be cubic, and the size thereof may be 5 millimeters × 5 millimeters. Tissue blocks included in the defined target region are selected for further calculation.

In one embodiment, the cancer probability calculation unit 1701 counts the number of malignant blocks (as the result of the classification) within the selected tissue block, to determine the cancer probability. The cancer probability may be determined as: an absolute number of malignant blocks (for example, 6 in the case of the target region 1800 in Fig. 19(A)); or the number obtained by dividing the number of malignant blocks by the total number of blocks within the target region (6/25 = 0.04 in the case of the target region 1800 in Fig. 19(A)).

In another embodiment, the cancer probability calculation unit 1701 considers the distribution of the malignant blocks. For example, a continuous cluster of malignant blocks may be calculated as having higher cancer probability than a randomly distributed cluster.

This cancer probability can be considered as the output of the tumor localization unit 750, and probability values may be displayed as color images so as to facilitate the examination. In another embodiment, the thresholding unit 1702 selects a threshold, and the probability calculated by the cancer probability calculation unit 1701 is compared with the threshold, so as to determine which tissue point is cancerous and which tissue point is normal. The tumor position is defined as an aggregate of cancerous tissue points based on the result.

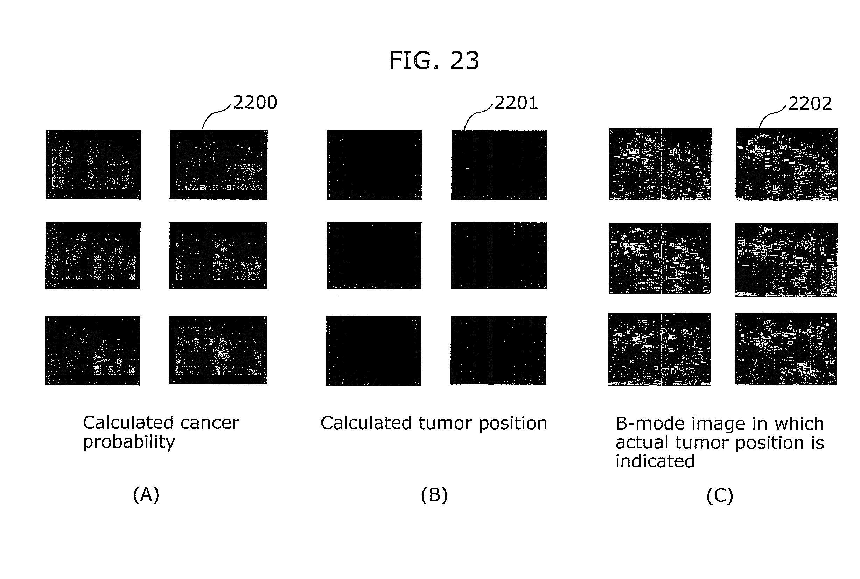

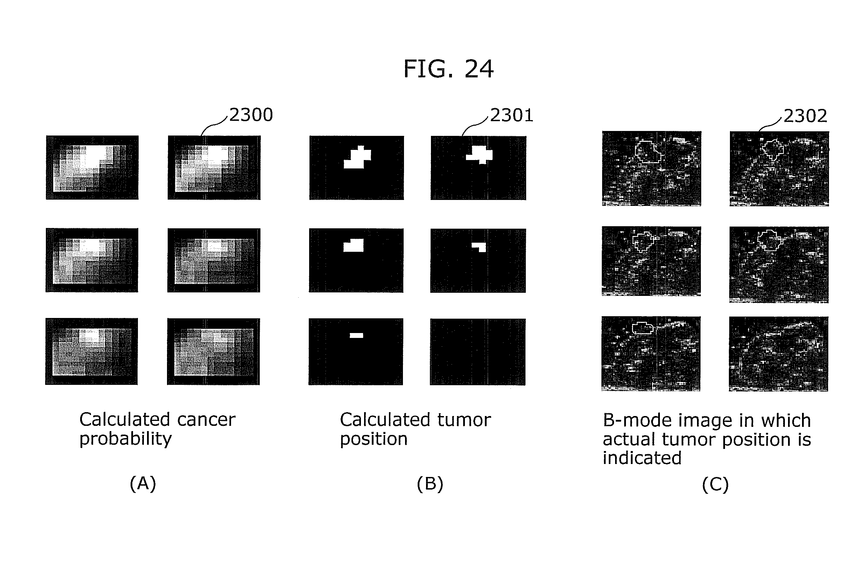

Figs. 23 and 24 show the results of an animal experiment performed to evaluate the performance of the tissue malignant tumor detection apparatus 90 according to the present invention. Each of the figures shows six consecutive ultrasound scan planes at 1-millimeter intervals. For each scan plane, the calculated cancer probability, a threshold result, and a corresponding B-mode image are displayed.

Fig. 23 shows the result when there is no tumor. An image 2200 shown in Fig. 23(A) indicates the cancer probability calculated using the method according to the embodiment of the present invention, indicating higher cancer probability in white and lower cancer probability in black. An image 2201 shown in Fig. 23(B) indicates a position of the tumor after thresholding, indicating the position of the cancerous tumor in white. An image 2202 shown in Fig. 23(C) shows an actual B-mode image. From the images 2200 and 2201, the tissue malignant tumor detection apparatus 90 according to the embodiment of the present invention can correctly indicate that there is no tumor in the tissue.

Fig. 24 shows the result when there is a tumor, and the actual position of the tumor is indicated by a white circle in an image 2302 in Fig. 24(C). Likewise, the image 2300 shown in Fig. 24(C) indicates the calculated cancer probability, and the image 2301 shown in Fig. 24(C) indicates the position of the tumor after thresholding. These images show that the tissue malignant tumor detection apparatus 90 according to the embodiment of the present invention can correctly indicate the position of the tumor in the tissue.

As described above, the inventive steps described above in the present Description are intended for determining the malignancy of the scanned tissue. It is possible to consider, but not limited to, using such information to determine, without previous knowledge of the scanned tissue, the position having higher probability of tumor existence in the tissue or the malignancy of the selected tissue region. Such information can also be used in parallel with or derived from another method for such usage, or can also be used to derive such another method. Such another method includes: ultrasound B-mode image, ultrasound Doppler imaging, elastography, computed tomography (CT), magnetic resonance imaging (MRI), positron emission tomography (PET), and photoacoustic tomography (PAT). Implementations in which the malignancy of the scanned tissue is determined using a combination of the inventive steps described in the present Description and another method are still within the scope of the present invention.

An example of such implementations is to identify a tumor mass in the scanned tissue using another method, and determine the malignancy of the identified tumor mass according to the present invention.

Note that in the present embodiment, the pulsation-related feature is at least one of: the pulsation amplitude feature, the pulsation shape feature, and the pulsation temporal feature. It is preferable that the pulsation-related feature include at least the pulsation shape feature or the pulsation temporal feature. This is because, as described earlier, it is possible to detect the malignant tumor with higher accuracy by considering the features derived from the irregularity of tissue pulsations in malignant tumors.

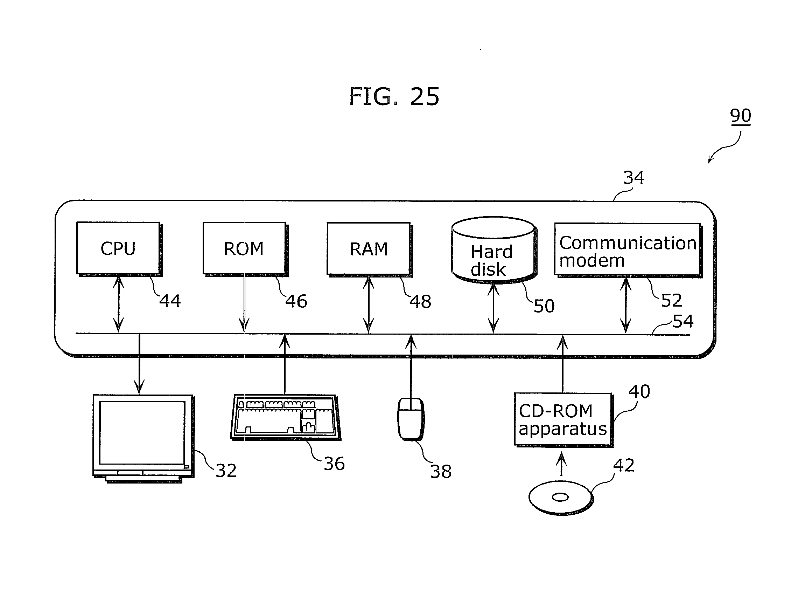

Note that the tissue malignant tumor detection apparatus 90 described in the present embodiment can also be realized by a computer. Fig. 25 is a block diagram showing a hardware configuration of a computer system which realizes the tissue malignant tumor detection apparatus 90.

The tissue malignant tumor detection apparatus 90 includes: a computer 34, a keyboard 36 and a mouse 38 with which to instruct the computer 34; a display 32 to present information such as calculation results by the computer 34; a compact disc read only memory (CD-ROM) apparatus 40 to read the program executed by the computer 34; and a communication modem (not shown).

The program that is processing performed by the tissue malignant tumor detection apparatus 90 is stored on a CD-ROM 42 that is a computer-readable medium, and is read by the CD-ROM apparatus 40. Alternatively, the program is read by the communication modem 52 through the computer network.

The computer 34 includes: a central processing unit (CPU) 44; a read only memory (ROM) 46; a random access memory (RAM) 48; a hard disk 50; a communication modem 52; and a bus 54.

The CPU 44 executes the program read via the CD-ROM apparatus 40 or the communication modem 52. The ROM 46 stores the program and data necessary for operating the computer 34. The RAM 48 stores the data such as a parameter for executing the program. The hard disk 50 stores the program and data. The communication modem 52 performs communication with another computer via the computer network 26. The bus 54 connects, mutually, the CPU 44, the ROM 46, the RAM 48, the hard disk 50, the communication modem 52, the display 32, the keyboard 36, the mouse 38, and the CD-ROM apparatus 40.

Furthermore, part or all of the constituent elements of each of the devices described above may be configured with one system large scale integration (LSI). The system LSI is a super multifunctional LSI manufactured by integrating a plurality of components on a single chip, and is specifically a computer system configured including a microprocessor, a ROM, a RAM, and so on. The RAM holds the computer program. The system LSI performs its function, with the microprocessor operating according to the computer program.

Furthermore, part or all of the constituent elements of each of the devices described above may include an IC card or single module that is detachable from and attachable to each device. The IC card or module is a computer system configured with a microprocessor, a ROM, a RAM, and so on. The IC card or module may include the super multifunctional LSI described above. The IC card or module performs its function, with the microprocessor operating according to the computer program. This IC card or module may have tamper resistance.

In addition, the present invention may be realized as the methods described above. In addition, these methods may be a computer program executing these methods through a computer, or may be a digital signal representing the computer program.

Furthermore, the present invention may be realized by recording the computer program or digital signal on a computer-readable recording medium that is, for example, a flexible disk, a hard disk, a CD-ROM, a magnetooptic disc (MO), a Digital Versatile Disc (DVD), a Digital Versatile Disc Read Only Memory (DVD-ROM), a Digital Versatile Disc Random Access Memory (DVD-RAM), a Blu-ray disc (BD) (registered trademark), a universal serial bus (USB) memory, a memory card such as a secure digital (SD) card, a semiconductor memory, and so on. In addition, the present embodiment may be realized as the digital signal recorded on these recording media.

In addition, the present invention may be realized as the computer program or digital signal transmitted via a telecommunications line, a wireless or wired communication line, a network represented by the Internet, data broadcasting and so on.

In addition, the present invention may be realized as a computer system including a microprocessor and a memory, and the memory may store the computer program, and the microprocessor may operate according to the computer program described above.

In addition, the program or the digital signal may be executed by another independent computer system by recording and transferring the program or the digital signal on the recording medium, or transferring the program or the digital signal via the network.

Furthermore, the embodiments and variations above may be combined with each other.

The embodiments described above should be considered to be not limitative but illustrative in all respects. It is intended that the scope of the present invention is represented not by the description above but by the claims, and all the variations and modifications are possible within the meaning and scope equivalent to the claims of the present invention.

[Industrial Applicability]

The present invention is applicable to a tissue malignant tumor detection method and so on, and is particularly applicable to a tissue malignant tumor detection method using ultrasound.

[Reference Signs List]

| | |
|---|---|
| 32 | Display |
| 34 | Computer |
| 36 | Keyboard |
| 38 | Mouse |
| 40 | CD-ROM apparatus |
| 42 | CD-ROM |
| 44 | CPU |
| 46 | ROM |
| 48 | RAM |
| 50 | Hard disk |
| 52 | Communication modem |
| 54 | Bus |
| 90 | Tissue malignant tumor detection apparatus |
| 100 | Block segmentation unit |
| 101 | Tissue pulsation estimation unit |
| 102 | Pre-processing unit |
| 103 | Pulsation-related feature extraction unit |
| 104 | Distribution characteristics calculation unit |
| 105 | Malignancy classification unit |
| 200 | Tissue displacement estimation unit |
| 201 | Tissue skewness estimation unit |
| 400 | Noise filtering unit |
| 401 | Major comportment extraction unit |
| 500 | Cardiac power calculation unit |
| 501 | Cardiac pulsation clustering unit |
| 502 | Extrema identification unit |
| 503 | Pulsation adjustment unit |
| 700 | Pulsation adjustment calculation unit |
| 701 | Pulsation adjustment calculation unit |
| 710 | Amplitude feature calculation unit |
| 711 | Shape feature calculation unit |
| 731 | Cardiac cycle delay calculation unit |
| 732 | Autoregressive coefficient calculation unit |
| 740 | Spatial distribution calculation unit |
| 750 | Tumor localization unit |
| 800 | Cardiac cycle identification unit |
| 801 | Shape feature extraction unit |
| 900 | Cardiac pulsation duration calculation unit |

901 Critical point identification unit
1300 Cardiac pulsation selection unit
1301 Pulsation tapering unit
1302 Representative pulsation extraction unit
1500 Gray-level co-occurrence matrix calculation unit
1501 Temporal feature calculation unit
1700 Tumor block segmentation unit
1701 Cancer probability calculation unit
1702 Thresholding unit
1900 Reference cardiac cycle determination unit
1901 Delay calculation unit
2000 Reference cardiac waveform calculation unit
2001 Individual difference calculation unit
2002 Total difference calculation unit
2100 Pulsation resampling unit
2101 Autoregressive formula calculation unit

**Claims**

1. A tissue malignant tumor detection method for detecting a malignant tumor included in a tissue, using a scan signal obtained by scanning the tissue with ultrasound, said tissue malignant tumor detection method comprising:

   segmenting a scanned region of the tissue into a plurality of blocks;
   estimating a tissue pulsation for each of the blocks, based on the scan signal, the tissue pulsation being a temporal variation in displacement of the tissue caused by pulsation of the tissue;
   extracting a plurality of pulsation-related features for each of the blocks, the pulsation-related features being parameters related to the tissue pulsation;
   calculating distribution characteristics of the pulsation-related features for each of the blocks; and
   classifying, based on the distribution characteristics, whether or not each of the blocks is a malignant block that is a block including a malignant tumor.

2. The tissue malignant tumor detection method according to Claim 1, further comprising
   locating a position of the malignant tumor, based on a block classified as the malignant block in said classifying.

3. The tissue malignant tumor detection method according to Claim 1,
   wherein said estimating includes:

   calculating a tissue displacement using the scan signal, the tissue displacement being a displacement of the tissue in spatial position;
   calculating tissue skewness from the calculated tissue displacement, the tissue skewness being a spatial gradient of the tissue displacement; and
   generating a pulsation waveform that is a waveform of the tissue pulsation and indicates the tissue displacement versus time or the tissue skewness versus time.

4. The tissue malignant tumor detection method according to Claim 1, further comprising
   extracting, from the estimated tissue pulsation, a component due to a cardiac pulsation derived from pulsations of a heart.

5. The tissue malignant tumor detection method according to Claim 4,
   wherein said extracting of a component further includes:

   calculating, of the estimated tissue pulsation, cardiac power that is electric power related to the cardiac pulsation;
   clustering the scanned region of the tissue, based on a magnitude of the cardiac power;
   identifying an extremum of an amplitude of the cardiac pulsation in the scanned region of the tissue that is clustered; and
   adjusting an amplitude of a pulsation waveform that is a waveform of the tissue pulsation, based on the extremum.

**6.** The tissue malignant tumor detection method according to Claim 5,
wherein said identifying of an extremum further includes:

identifying peaks and troughs in the pulsation waveform; and
rejecting an outlier in the amplitude of the pulsation waveform, so as to eliminate an interference peak and an interference trough, the interference peak being a peak with which a gap from another peak is larger than a predetermined threshold, and the interference trough being a trough with which a gap from another trough is larger than a predetermined threshold.

**7.** The tissue malignant tumor detection method according to Claim 5,
wherein said extracting of a component further includes:

eliminating portions related to an interference peak and an interference trough, the interference peak being a peak with which a gap from another peak is larger than a predetermined threshold, and an interference trough being a trough with which a gap from another trough is larger than a predetermined threshold; and
adjusting the amplitude of the pulsation waveform so that the peaks in the pulsation waveform are aligned in a temporal direction and the troughs in the pulsation waveform are aligned in the temporal direction.

**8.** The tissue malignant tumor detection method according to Claim 1,
wherein in said estimating, the tissue pulsation is estimated for all scan points in each of the blocks.

**9.** The tissue malignant tumor detection method according to Claim 1,
wherein in said estimating, the tissue pulsation is estimated as at least one representative pulsation for each of the blocks or a combination of the at least one representative pulsation.

**10.** The tissue malignant tumor detection method according to Claim 1,
wherein the pulsation-related features include at least one of: a pulsation amplitude feature that is a feature quantity related to an amplitude of the tissue pulsation; a pulsation shape feature that is a feature quantity related to a shape of a waveform of the tissue pulsation; and a pulsation temporal feature that is a feature quantity related to a temporal variation of the waveform of the tissue pulsation.

**11.** The tissue malignant tumor detection method according to Claim 10,
wherein the pulsation shape feature is at least one of:

L1/L2 which is a ratio between L1 that is a systolic duration and L2 that is a diastolic duration, the systolic duration being a duration of a systolic part of a cardiac cycle, and the diastolic duration being a duration of a diastolic part of the cardiac cycle;
L3/L4 which is a ratio between L3 that is a period from a systolic mid-point to a diastolic mid-point and L4 that is a duration of a cardiac pulsation, the systolic mid-point being a point at which, on a systolic curve, a ratio of the amplitude with respect to a maximum amplitude equals a predetermined ratio in the systolic duration, and the diastolic mid-point being a point at which, on a diastolic curve, a ratio of the amplitude with respect to the maximum amplitude equals a predetermined ratio in the diastolic duration;
a deviation of the diastolic curve from a predetermined curve connecting a systolic peak that is a peak in the amplitude in the systolic duration and a diastolic ending point that is an ending point of the diastolic duration;
skewness which represents asymmetry of the cardiac pulsation;
kurtosis which represents peakedness of the systolic peak; and
a deviation of an extremum included in the diastolic curve.

**12.** The tissue malignant tumor detection method according to Claim 10,
wherein the pulsation shape feature is calculated in:

calculating a cardiac cycle from the tissue pulsation;
identifying a critical point using the cardiac cycle; and
extracting the pulsation shape feature based on the cardiac cycle and the critical point.

**13.** The tissue malignant tumor detection method according to Claim 12,
wherein the critical point of the cardiac cycle includes: a systolic starting point which is a starting point of a systolic part of the cardiac cycle; a diastolic ending point which is an ending point of a diastolic part of the cardiac cycle; a systolic peak which is a peak in the amplitude during the systolic part; a systolic mid-point which is a point at which, on a systolic curve, a ratio of the amplitude with respect to a maximum amplitude equals a predetermined ratio during the systolic part of the cardiac cycle; and a diastolic mid-point which is a point at which, on a diastolic curve, a ratio of the amplitude with respect to a maximum amplitude equals a predetermined ratio during the diastolic part of the cardiac cycle.

14. The tissue malignant tumor detection method according to Claim 12,
wherein said identifying of a critical point includes:

searching a minimum point and a maximum point in the tissue pulsation;
identifying a pulsation direction which indicates whether the tissue pulsation has an upward systolic curve or a downward systolic curve, based on the minimum point and the maximum point; and
determining the critical point in the pulsation waveform, using the maximum point, the minimum point, and the pulsation direction.

15. The tissue malignant tumor detection method according to Claim 11,
wherein the predetermined curve is linear.

16. The tissue malignant tumor detection method according to Claim 11,
wherein in said extracting of pulsation-related features, the deviation is calculated as a sum of positive differences between the diastolic curve and the predetermined curve when the pulsation has an upward systolic curve, and the deviation is calculated as a sum of negative differences between the diastolic curve and the predetermined curve when the pulsation has a downward systolic curve.

17. The tissue malignant tumor detection method according to Claim 10,
wherein the pulsation temporal feature is at least one of: a delay of a cardiac cycle; a difference in a cardiac waveform that is a waveform of a cardiac pulsation; and an autoregressive coefficient of a waveform of the tissue pulsation.

18. The tissue malignant tumor detection method according to Claim 17,
wherein the delay of the cardiac cycle is calculated in calculating of the delay of the cardiac cycle, which includes:

determining a reference cardiac cycle that is a cardiac cycle to be referenced; and
calculating a delay of a target cardiac cycle with respect to the reference cardiac cycle.

19. The tissue malignant tumor detection method according to Claim 18,
wherein in said determining of a reference cardiac cycle, a cardiac cycle having a largest amplitude among scan data, is selected as the reference cardiac cycle.

20. The tissue malignant tumor detection method according to Claim 18,
wherein the reference cardiac cycle is determined from an electrocardiography (ECG) signal.

21. The tissue malignant tumor detection method according to Claim 19,
wherein the difference in the cardiac waveform is calculated in said calculating of a delay of a target cardiac cycle, which includes:

calculating a reference cardiac waveform that is a cardiac waveform to be referenced;
calculating a difference between each of a plurality of cardiac waveforms derived from the pulsation and the reference cardiac waveform; and
calculating a total cardiac waveform difference value from a plurality of differences each of which is the calculated difference, the total cardiac waveform difference value being a value representing the differences between the cardiac waveforms and the reference cardiac waveform.

22. The tissue malignant tumor detection method according to Claim 21,
wherein the total cardiac waveform difference value is a standard deviation of the calculated differences.

23. The tissue malignant tumor detection method according to Claim 17,
wherein the autoregressive coefficient is calculated in calculating the autoregressive coefficient, which includes:

tapering a plurality of pulsation waveforms to have the same cardiac cycle; and
calculating the autoregressive coefficient which is a coefficient of a predetermined autoregressive model, based on the predetermined autoregressive model and the tapered pulsation waveforms.

**24.** The tissue malignant tumor detection method according to Claim 1,
wherein the distribution characteristics are at least one of a spatial distribution parameter and a statistical distribution parameter.

**25.** The tissue malignant tumor detection method according to Claim 24,
wherein the spatial distribution parameter is at least one of energy, entropy, contrast, homogeneity, and correlation of the pulsation-related features.

**26.** The tissue malignant tumor detection method according to Claim 24,
wherein the statistical distribution parameter is at least one of a mean, a median, a largest value, a smallest value, a standard deviation, kurtosis, and skewness of the pulsation-related features.

**27.** The tissue malignant tumor detection method according to Claim 1,
wherein the pulsation-related features and the distribution characteristics of the pulsation-related features include at least one of:

a median, entropy, a standard deviation, a mean, and a largest value of the pulsation amplitude at a plurality of scan points in each of the blocks;
a median, entropy, a standard deviation, a mean, and a largest value of a cardiac waveform difference at the scan points in each of the blocks;
a median of a ratio between a systolic duration and a diastolic duration at the scan points in each of the blocks, the systolic duration being a duration of a systolic part of a cardiac cycle, and the diastolic duration is being a duration of a diastolic part of the cardiac cycle;
a ratio between the systolic duration and the diastolic duration of a representative pulsation in each of the blocks; and
a largest deviation value and a standard deviation of a diastolic curve at the scan points in each of the blocks.

**28.** The tissue malignant tumor detection method according to Claim 2,
wherein said locating further includes:

setting a target region for each of scan points in the scanned tissue;
identifying a block belonging to the target region, from among the blocks; and
calculating a probability of the tissue being a cancer, based on a result of said classifying performed on the block belonging to the target region.

**29.** The tissue malignant tumor detection method according to Claim 28,
wherein said locating further includes:

displaying, in two or three dimensional image, the probability of the tissue being a cancer at the scan points in the scanned tissue.

**30.** A tissue malignant tumor detection apparatus which detects a malignant tumor included in a tissue, using a scan signal obtained by scanning the tissue with ultrasound, said tissue malignant tumor detection apparatus comprising:

a block segmentation unit configured to segment a scanned region of the tissue into a plurality of blocks;
a tissue pulsation estimation unit configured to estimate a tissue pulsation for each of the blocks based on the scan signal, the tissue pulsation being a temporal variation in displacement of the tissue caused by pulsation of the tissue;
a pulsation-related feature extraction unit configured to extract pulsation-related features for each of the blocks, the pulsation-related features being parameters related to the tissue pulsation;
a distribution characteristics calculation unit configured to calculate distribution characteristics of the pulsation-related features for each of the blocks; and
a malignancy classification unit configured to classify, based on the distribution characteristics, whether or not each of the blocks is a malignant block that is a block including a malignant tumor.

**31.** The tissue malignant tumor detection apparatus according to Claim 30, further comprising
a tumor localization unit configured to locate a position of the malignant tumor, based on a block classified as the malignant block by said malignancy classification unit.

**Amended claims under Art. 19.1 PCT**

**1. (Amended)** A tissue malignant tumor detection apparatus which detects a malignant tumor included in a tissue, using a scan signal obtained by scanning the tissue with ultrasound, said tissue malignant tumor detection apparatus comprising :

a block segmentation unit configured to segment a scanned region of the tissue into a plurality of blocks;
a tissue pulsation estimation unit configured to estimate a tissue pulsation for each of the blocks, based on the scan signal, the tissue pulsation being a temporal variation in displacement of the tissue caused by pulsation of the tissue;
a pulsation-related feature extraction unit configured to extract a plurality of pulsation-related features for each of the blocks, the pulsation-related features being parameters related to the tissue pulsation;
a distribution characteristics calculation unit configured to calculate distribution characteristics of the pulsation-related features for each of the blocks; and
a malignancy classification unit configured to classify, based on the distribution characteristics, whether or not each of the blocks is a malignant block that is a block including a malignant tumor.

**2. (Amended)** The tissue malignant tumor detection apparatus according to Claim 1,
wherein said malignancy classification unit includes a tumor localization unit configured to locate a position of the malignant tumor, based on a block classified as the malignant block.

**3. (Amended)** The tissue malignant tumor detection apparatus according to Claim 1,
wherein said tissue pulsation estimation unit includes:

a tissue displacement calculation unit configured to
calculate a tissue displacement using the scan signal, the tissue displacement being a displacement of the tissue in spatial position;
a tissue skewness calculation unit configured to calculate tissue skewness from the calculated tissue displacement, the tissue skewness being a spatial gradient of the tissue displacement; and
a pulsation waveform generation unit configured to generate a pulsation waveform that is a waveform of the tissue pulsation and indicates the tissue displacement versus time or the tissue skewness versus time.

**4. (Amended)** The tissue malignant tumor detection apparatus according to Claim 1, further comprising
a pre-processing unit configured to extract, from the estimated tissue pulsation, a component due to a cardiac pulsation derived from pulsations of a heart.

**5. (Amended)** The tissue malignant tumor detection apparatus according to Claim 4,
wherein said pre-processing unit further includes:

a cardiac power calculation unit configured to calculate, of the estimated tissue pulsation, cardiac power that is electric power related to the cardiac pulsation;
a cardiac pulsation clustering unit configured to cluster the scanned region of the tissue, based on a magnitude of the cardiac power;
an extrema identification unit configured to identify an extremum of an amplitude of the cardiac pulsation in the scanned region of the tissue that is clustered; and
a cardiac pulsation adjustment unit configured to adjust an amplitude of a pulsation waveform that is a waveform of the tissue pulsation, based on the extremum.

**6. (Amended)** The tissue malignant tumor detection apparatus according to Claim 5,
wherein said extrema identification unit further includes:

an extremum point identification unit configuration to identify peaks and troughs in the pulsation waveform; and
an outlier rejection unit configured to reject an outlier in the amplitude of the pulsation waveform, so as to eliminate an interference peak and an interference trough, the interference peak being a peak with which a gap from another peak is larger than a predetermined threshold, and the interference trough being a trough with which a gap from another trough is larger than a predetermined threshold.

**7.** (Amended) The tissue malignant tumor detection apparatus according to Claim 5, wherein said pre-processing unit further includes:

an interference elimination unit configured to eliminate portions related to an interference peak and an interference trough, the interference peak being a peak with which a gap from another peak is larger than a predetermined threshold, and an interference trough being a trough with which a gap from another trough is larger than a predetermined threshold; and
a pulsation adjustment unit configured to adjust the amplitude of the pulsation waveform so that the peaks in the pulsation waveform are aligned in a temporal direction and the troughs in the pulsation waveform are aligned in the temporal direction.

**8.** (Amended) The tissue malignant tumor detection apparatus according to Claim 1, wherein said tissue pulsation estimation unit is configured to estimate the tissue pulsation for all scan points in each of the blocks.

**9.** (Amended) The tissue malignant tumor detection apparatus according to Claim 1, wherein said tissue pulsation estimation unit is configured to estimate the tissue pulsation as at least one representative pulsation for each of the blocks or a combination of the at least one representative pulsation.

**10.** (Amended) The tissue malignant tumor detection apparatus according to Claim 1, wherein the pulsation-related features include at least one of: a pulsation amplitude feature that is a feature quantity related to an amplitude of the tissue pulsation; a pulsation shape feature that is a feature quantity related to a shape of a waveform of the tissue pulsation; and a pulsation temporal feature that is a feature quantity related to a temporal variation of the waveform of the tissue pulsation.

**11.** (Amended) The tissue malignant tumor detection appararus according to Claim 10, wherein the pulsation shape feature is at least one of:

L1/L2 which is a ratio between L1 that is a systolic duration and L2 that is a diastolic duration, the systolic duration being a duration of a systolic part of a cardiac cycle, and the diastolic duration being a duration of a diastolic part of the cardiac cycle;
L3/L4 which is a ratio between L3 that is a period from a systolic mid-point to a diastolic mid-point and L4 that is a duration of a cardiac pulsation, the systolic mid-point being a point at which, on a systolic curve, a ratio of the amplitude with respect to a maximum amplitude equals a predetermined ratio in the systolic duration, and the diastolic mid-point being a point at which, on a diastolic curve, a ratio of the amplitude with respect to the maximum amplitude equals a predetermined ratio in the diastolic duration;
a deviation of the diastolic curve from a predetermined curve connecting a systolic peak that is a peak in the amplitude in the systolic duration and a diastolic ending point that is an ending point of the diastolic duration;
skewness which represents asymmetry of the cardiac pulsation;
kurtosis which represents peakedness of the systolic peak; and
a deviation of an extremum included in the diastolic curve.

**12.** (Amended) The tissue malignant tumor detection apparatus according to Claim 10, wherein the pulsation shape feature is calculated by a shape feature calculation unit which includes:

a cardiac pulsation duration calculation unit configured to). calculate a cardiac cycle from the tissue pulsation;
a critical point identification unit configured to identify a critical point using the cardiac cycle; and
a shape feature extraction unit configured to extract the pulsation shape feature based on the cardiac cycle and the critical point.

**13.** (Amended) The tissue malignant tumor detection apparatus according to Claim 12, wherein the critical point of the cardiac cycle includes: a systolic starting point which is a starting point of a systolic part of the cardiac cycle; a diastolic ending point which is an ending point of a diastolic part of the cardiac cycle; a systolic peak which is a peak in the amplitude during the systolic part; a systolic mid-point which is a point at which, on a systolic curve, a ratio of the amplitude with respect to a maximum amplitude equals a predetermined ratio during the systolic part of the cardiac cycle; and a diastolic mid-point which is a point at which, on a diastolic curve, a ratio of the amplitude with respect to a maximum amplitude equals a predetermined ratio during the diastolic part of the cardiac cycle.

**14.** (Amended) The tissue malignant tumor detection apparatus according to Claim 12,
wherein said identification unit includes:

a search unit configured to search a minimum point and a maximum point in the tissue pulsation;
a pulsation identification unit configured to identify a pulsation direction which indicates whether the tissue pulsation has an upward systolic curve or a downward systolic curve, based on the minimum point and the maximum point; and
a critical point determination unit configured to determine the critical point in the pulsation waveform, using the maximum point, the minimum point, and the pulsation direction.

**15.** (Amended) The tissue malignant tumor detection apparatus according to Claim 11,
wherein the predetermined curve is linear.

**16.** (Amended) The tissue malignant tumor detection apparatus according to Claim 11,
wherein said pulsation-related feature extraction unit is configured to calculate the deviation as a sum of positive differences between the diastolic curve and the predetermined curve when the pulsation has an upward systolic curve, and calculate the deviation as a sum of negative differences between the diastolic curve and the predetermined curve when the pulsation has a downward systolic curve.

**17.** (Amended) The tissue malignant tumor detection apparatus according to Claim 10,
wherein the pulsation temporal feature is at least one of: a delay of a cardiac cycle; a difference in a cardiac waveform that is a waveform of a cardiac pulsation; and an autoregressive coefficient of a waveform of the tissue pulsation.

**18.** (Amended) The tissue malignant tumor detection apparatus according to Claim 17,
wherein the delay of the cardiac cycle is calculated by a cardiac cycle delay calculation unit which includes:
a reference cardiac cycle determination unit configured to determine a reference cardiac cycle that is a cardiac cycle to be referenced; and
a delay calculation unit configured to calculate a delay of a target cardiac cycle with respect to the reference cardiac cycle.

**19.** (Amended) The tissue malignant tumor detection apparatus according to Claim 18,
wherein said reference cardia cycle determination unit is configured to select a cardiac cycle having a largest amplitude among scan data, as the reference cardiac cycle.

**20.** (Amended) The tissue malignant tumor detection apparatus according to Claim 18,
wherein the reference cardiac cycle is determined from an electrocardiography (ECG) signal.

**21.** (Amended) The tissue malignant tumor detection apparatus according to Claim 19,
wherein the difference in the cardiac waveform is calculated said delay calculation unit which includes:

a reference cardiac waveform calculation unit configured to calculate a reference cardiac waveform that is a cardiac waveform to be referenced;
an individual difference calculation unit configured to calculate a difference between each of a plurality of cardiac waveforms derived from the pulsation and the reference cardiac waveform; and
a total difference calculation unit configured to calculate a total cardiac waveform difference value from a plurality of differences each of which is the calculated difference, the total cardiac waveform difference value being a value representing the differences between the cardiac waveforms and the reference cardiac waveform.

**22.** (Amended) The tissue malignant tumor detection apparatus according to Claim 21,
wherein the total cardiac waveform difference value is a standard deviation of the calculated differences.

**23.** (Amended) The tissue malignant tumor detection apparatus according to Claim 17,
wherein the autoregressive coefficient is calculated

by an autogressive coefficient calculation unit which includes:
a pulsation resampling unit configured to taper a plurality of pulsation waveforms to have the same cardiac cycle; and
an autogressive formula calculation unit configured to calculate the autoregressive coefficient which is a coefficient of a predetermined autoregressive model, based on the predetermined autoregressive model and the tapered pulsation waveforms.

24. (Amended) The tissue malignant tumor detection apparatus according to Claim 1,
wherein the distribution characteristics are at least one of a spatial distribution parameter and a statistical distribution parameter.

25. (Amended) The tissue malignant tumor detection apparatus according to Claim 24,
wherein the spatial distribution parameter is at least one of energy, entropy, contrast, homogeneity, and correlation of the pulsation-related features.

26. (Amended) The tissue malignant tumor detection apparatus according to Claim 24,
wherein the statistical distribution parameter is at least one of a mean, a median, a largest value, a smallest value, a standard deviation, kurtosis, and skewness of the pulsation-related features.

27. (Amended) The tissue malignant tumor detection apparatus according to Claim 1,
wherein the pulsation-related features and the distribution characteristics of the pulsation-related features include at least one of:

a median, entropy, a standard deviation, a mean, and a largest value of the pulsation amplitude at a plurality of scan points in each of the blocks;
a median, entropy, a standard deviation, a mean, and a largest value of a cardiac waveform difference at the scan points in each of the blocks;
a median of a ratio between a systolic duration and a diastolic duration at the scan points in each of the blocks, the systolic duration being a duration of a systolic part of a cardiac cycle, and the diastolic duration is being a duration of a diastolic part of the cardiac cycle;
a ratio between the systolic duration and the diastolic duration of a representative pulsation in each of the blocks; and
a largest deviation value and a standard deviation of a diastolic curve at the scan points in each of the blocks.

28. (Amended) The tissue malignant tumor detection apparatus according to Claim 2,
wherein said tumor localization further includes:

a target region specification unit configured to set a target region for each of scan points in the scanned tissue;
a tumor block segmentation unit configured to identify a block belonging to the target region, from among the blocks; and
a cancer probability calculation unit configured to calculate a probability of the tissue being a cancer, based on a result of the classification performed by said malignancy classification unit on the block belonging to the target region.

29. (Amended) The tissue malignant tumor detection apparatus according to Claim 28,
wherein said tumor localization unit further includes:

an imaging unit configured to display, in two or three dimensional image, the probability of the tissue being a cancer at the scan points in the scanned tissue.

30. (Cancelled)

31. (Cancelled)

32. (New) A tissue malignant tumor detection method for detecting a malignant tumor included in a tissue, using a scan signal obained by scanning the tissue with ultrasound, said tissue malignant tumor detection method comprising :

segmenting a scanned region of the tissue into a plurality of blocks;

estimating a tissue pulsation for each of the blocks, based on the scan signal, the tissue pulsation being a temporal variation in displacement of the tissue caused by pulsation of the tissue;
extracting a plurality of pulsation-related features for each of the blocks, the pulsation-related features being parameters related to the tissue pulsation ;
calculating distribution characteristics of the pulsation-related features for each of the blocks; and
classifying, based on the distribution characteristics, whether or not each of the blocks is a malignant block that is a block including a malignant tumor.

**33.** (New) A program for causing a computer to execute the tissue malignant tumor detection method according to Claim 32.

# FIG. 1

Tissue malignant tumor detection unit — 90

scannedSig

Block segmentation unit — 100

Tissue pulsation estimation unit — 101

Pre-processing unit — 102

Pulsation-related feature extraction unit — 103

Distribution characteristics calculation unit — 104

Malignancy classification unit — 105

blocks

rawPulse

procPulse

pulseFeature

pulseFeature

distributionFeature

procPulse

maligScores

EP 2 578 163 A1

FIG. 2

```
              ┌─────────────┐
              │    Start     │
              └──────┬──────┘
                     │
                     ▼
          ┌──────────────────────┐    S100
          │  Segment scan region │
          └──────────┬───────────┘
                     │
                     ▼
          ┌──────────────────────┐    S101
          │ Estimate tissue pulsation │
          └──────────┬───────────┘
                     │
                     ▼
          ┌───────────────────────────┐   S103
          │ Extract pulsation-related features │
          └──────────┬────────────────┘
                     │
                     ▼
          ┌───────────────────────────┐   S104
          │ Calculate distribution characteristics │
          │ of pulsation-related features │
          └──────────┬────────────────┘
                     │
                     ▼
          ┌───────────────────────────┐   S105
          │ Classify according to malignancy │
          └──────────┬────────────────┘
                     │
                     ▼
              ┌─────────────┐
              │     End      │
              └─────────────┘
```

# FIG. 3

scannedSig(d,l,f) → **Tissue displacement estimation unit** (200) → disp(d,l,f) → **Tissue skewness estimation unit** (201) → rawPulse(d,l,f)

Tissue pulsation estimation unit (101)

EP 2 578 163 A1

# FIG. 4

Ultrasound transducer

Scan direction

Depth direction (fast time)

Line direction

Frame direction (slow time)

# FIG. 5

102

Pre-processing

rawPulse → [ 400 Noise filtering unit ] → [ 401 Major component extraction unit ] → procPulse

FIG. 6

Major component extraction unit — 401

rawPulse(d,l,f) → Cardiac power calculation unit (500) → cardiacPow(d,l) → Cardiac pulsation clustering unit (501) → cardiacCluster(d,l) → Extrema identification unit (502) → cardiacExtrema(d,l) → Pulsation adjustment unit (503)

cardiacCluster(d,l) → Pulsation adjustment unit (503)

rawPulse(d,l,f) → Pulsation adjustment unit (503)

Pulsation adjustment unit (503) → procPulse(d,l,f)

FIG. 7

(A) Time (second)
Pulsation amplification

(B) Time (second)
Pulsation amplification

(C) Time (second)
Pulsation amplification

(D) Time (second)
Pulsation amplification

FIG. 8

EP 2 578 163 A1

## FIG. 9

711

Shape feature
calculation unit

800

procPulse(d,l,f) → Cardiac cycle identification unit —criticalPoints(d,l)→ 801

Shape feature extraction unit → pulseShapeFeatures

# FIG. 10

procPulse(d,l,f)

800

Cardiac cycle
identification unit

900

Cardiac pulsation
duration
calculation unit

cardiacPeriod(d,l)

901

Critical point
identification unit

criticalPoints(d,l)

FIG. 11

# FIG. 12

(A) Upward pulsation

(B) Downward pulsation

FIG. 13

(A) Tumoral pulsation

1200

(B) Normal pulsation

1201

EP 2 578 163 A1

## FIG. 14

rawPulse(d,l,f)

~401

~1300   Major component
        extraction unit

Cardiac pulsation
selection unit

cardiacPulse(d,l,f)

~1301

Pulsation
tapering unit

taperedCardiacPulse(d,l,f)

~1302

Representative
pulsation
extraction unit

procPulse(d,l,f)

## FIG. 15

(A) Segmented cardiac pulsation — 1400

(B) Customized Hann window — 1401

(C) Tapered cardiac pulsation — 1402

(D) Average cardiac pulsation — 1403

EP 2 578 163 A1

# FIG. 16

pulseFeature

740

Spatial distribution
calculation unit

1500

Gray-level
co-occurrence
matrix calculation
unit

offset

GLCM(offset)

1501

Spatial feature
calculation unit

spatialFeature

# FIG. 17

135°    90°    45°

1:Offset (i.e., direction and distance)

(A)

Target pixel → 0°

2:Calculate gray-level co-occurrence matrix per offset (GLCM)

Offset d= [0, 1]

(B)

| 1 | 1 | 5 | 6 | 8 |
|---|---|---|---|---|
| 2 | 3 | 5 | 7 | 1 |
| 4 | 5 | 7 | 1 | 2 |
| 8 | 5 | 1 | 2 | 5 |

Feature value normalized in 8 gray levels in block

(C)

j →

| | 1 | 2 | 3 | 4 | 5 | 6 | 7 | 8 |
|---|---|---|---|---|---|---|---|---|
| 1 | 1 | 2 | 0 | 0 | 1 | 0 | 0 | 0 |
| 2 | 0 | 0 | 1 | 0 | 1 | 0 | 0 | 0 |
| 3 | 0 | 0 | 0 | 0 | 1 | 0 | 0 | 0 |
| 4 | 0 | 0 | 0 | 0 | 1 | 0 | 0 | 0 |
| 5 | 1 | 0 | 0 | 0 | 0 | 1 | 2 | 0 |
| 6 | 0 | 0 | 0 | 0 | 0 | 0 | 0 | 1 |
| 7 | 2 | 0 | 0 | 0 | 0 | 0 | 0 | 0 |
| 8 | 0 | 0 | 0 | 0 | 1 | 0 | 0 | 0 |

i ↓

GLCM: value Pd(i, j) in i-th row j-th column represents transition number from gray level value i to gray level j in offset direction

EP 2 578 163 A1

## FIG. 18

maligScores

750

Tumor localization unit

1700

Tumor block segmentation unit

block(d,l) →

1701

Cancer probability calculation unit

cancerProb(d,l)

1702

Thresholding unit

tumorLoc

## FIG. 19

Result of block classification
Black: malignant
White: normal

Count the number of malignant blocks in target region
→ Cancer probability

Probability: 6/25

1800

1801

Probability: 3/25

2D

(A)

Cancer probability: count malignant blocks across entire selected scan planes

5th plane

5mm

1st plane

3D

(B)

# FIG. 20

procPulse(d,l,f)

~731

Cardiac cycle delay
calculation unit

~1900

Reference cardiac
cycle determination
unit

refCarCycle(f)

~1901

Delay calculation
unit

carDelay(d,l)

**FIG. 21**

procPulse(d,l,f)

731

Cardiac cycle delay calculation unit

2000

Reference cardiac waveform calculation unit

refCarWaveform(f)

1901

Delay calculation unit

2001

Individual difference calculation unit

indivDiff(f)

2002

Total difference calculation unit

carDiff(d,l)

EP 2 578 163 A1

# FIG. 22

procPulse(d,l,f)

732

Autoregressive coefficient
calculation unit

2100

Pulsation
resampling unit

resampPulse(d,l,f)

2101

arModel →

Autoregressive
formula calculation
unit

arCoeffs(d,l)

# FIG. 23

Calculated cancer
probability

(A)

Calculated tumor position

(B)

B-mode image in which
actual tumor position is
indicated

(C)

EP 2 578 163 A1

# FIG. 24

Calculated cancer
probability

(A)

Calculated tumor
position

(B)

B-mode image in which
actual tumor position is
indicated

(C)

# FIG. 25

CPU  44

ROM  46

RAM  48

Hard disk  50

Communication modem  52

34

90

54

32

36

38

CD-ROM apparatus  40

42

EP 2 578 163 A1

## INTERNATIONAL SEARCH REPORT

| International application No. |
|---|
| PCT/JP2011/003156 |

**A. CLASSIFICATION OF SUBJECT MATTER**
*A61B8/08*(2006.01)i, *A61B8/02*(2006.01)i

According to International Patent Classification (IPC) or to both national classification and IPC

**B. FIELDS SEARCHED**

Minimum documentation searched (classification system followed by classification symbols)
A61B8/08, A61B8/02

Documentation searched other than minimum documentation to the extent that such documents are included in the fields searched
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 2010-51554 A (Konica Minolta Medical & Graphic, Inc.), 11 March 2010 (11.03.2010), entire text; all drawings (Family: none) | 30,31 |
| A | JP 2010-512900 A (Institut Gustave Roussy -IGR-), 30 April 2010 (30.04.2010), entire text; all drawings & US 2010/0060644 A & EP 2097007 A & WO 2008/053268 A1 | 30,31 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 June, 2011 (17.06.11) | 28 June, 2011 (28.06.11) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2011/003156

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2009-183564 A  (Hitachi Medical Corp.),<br>20 August 2009 (20.08.2009),<br>entire text; all drawings<br>& US 2010/0331694 A      & EP 2243431 A1<br>& WO 2009/098961 A1      & CN 101938942 A | 30,31 |
| A | JP 2003-116855 A  (Aloka Co., Ltd.),<br>22 April 2003 (22.04.2003),<br>entire text; all drawings<br>(Family: none) | 30,31 |

Form PCT/ISA/210 (continuation of second sheet) (July 2009)

## INTERNATIONAL SEARCH REPORT

International application No.

PCT/JP2011/003156

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 1-29
   because they relate to subject matter not required to be searched by this Authority, namely:
   Claims 1 to 29 pertain to methods for treatment of the human body by surgery or therapy and diagnostic methods and thus relate to a subject matter which this International Searching Authority is not required, under the provisions of PCT Article 17(2)(a)(i) and PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030149358 A
- US 20060241463 A
- US 5233994 A
- US 4276885 A
- US 8002365 B
- US 0128594 A
- US 20040199077 A
- US 6984211 B
- US 20050027188 A
- US 7466848 B